(19)

**Europäisches Patentamt**
**European Patent Office**
**Office européen des brevets**

(11)  **EP 4 567 149 A1**

(12)  **EUROPEAN PATENT APPLICATION**
published in accordance with Art. 153(4) EPC

(43) Date of publication:
**11.06.2025  Bulletin 2025/24**

(21) Application number: **23849408.2**

(22) Date of filing: **01.08.2023**

(51) International Patent Classification (IPC):
*C23C 8/26* (2006.01)    *C23C 8/36* (2006.01)
*C23C 8/22* (2006.01)    *C23C 8/06* (2006.01)

(52) Cooperative Patent Classification (CPC):
**C23C 8/06; C23C 8/22; C23C 8/26; C23C 8/36**

(86) International application number:
**PCT/CN2023/110535**

(87) International publication number:
**WO 2024/027702 (08.02.2024 Gazette 2024/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.08.2022  CN 202210916370**
**07.09.2022  CN 202211091239**

(71) Applicant: **Biotyx Medical (Shenzhen) Co., Ltd.**
**Shenzhen, Guangdong 518000 (CN)**

(72) Inventors:
• **OU, Yetao**
  **Shenzhen, Guangdong 518000 (CN)**
• **SHI, Xiaoli**
  **Shenzhen, Guangdong 518000 (CN)**
• **ZHANG, Deyuan**
  **Shenzhen, Guangdong 518000 (CN)**
• **WANG, Wenbin**
  **Shenzhen, Guangdong 518000 (CN)**

(74) Representative: **Prinz & Partner mbB**
**Patent- und Rechtsanwälte**
**Rundfunkplatz 2**
**80335 München (DE)**

(54)  **METHOD FOR MODIFYING ENTIRE METAL WORKPIECE, AND GAS GUIDE ASSEMBLY AND DEVICE FOR MODIFICATION**

(57)    The present invention belongs to the technical field of medical instruments and particularly relates to a method for integrally modifying a metal workpiece, and a gas guide assembly and an apparatus for modification. The method includes the steps of placing the metal workpiece in a gas stream for modification, and cooling a modified metal workpiece, wherein in the step of modification, the gas stream is preheated to a near-modification temperature, and then flows onto a surface of the metal workpiece. The gas guide assembly includes a gas guide pipe, and a relational expression of a length L and an inner radius R of the gas guide pipe is E=LR²+A, 0 ≤ A ≤ 5 cm³ and 0.3 cm³ ≤ E ≤ 400 cm³. Through the above-mentioned relational expression, the values of the length and the inner radius of the gas guide pipe may be set, a non-metallic gas can be preheated to a reaction temperature by the gas guide pipe and then discharged, and the discharged gas may maintain a stable temperature and decomposition rate and a suitable flow rate to improve the uniformity of the non-metal permeating treatment of the metal workpiece. The apparatus of the present invention can perform large-batch metal workpiece modification treatment for prefabricated members, and a good metal workpiece modification treatment effect is achieved.

**FIG. 4a**

## Description

The present invention claims priority to Chinese Patent Application No. 202210916370.4 entitled "Method for Integrally Modifying Metal Workpiece" filed to China National Intellectual Property Administration on August 1, 2022 and Chinese Patent Application No. 202211091239.5 entitled "Non-metal Permeating Gas Guide Assembly, Non-metal Permeating Apparatus, and Non-metal Permeating Method" filed to China National Intellectual Property Administration on September 7, 2022, the entire contents of which are incorporated herein by reference.

## Technical Field

The present invention belongs to the technical field of medical instruments, and particularly relates to a method for integrally modifying a metal workpiece, and a gas guide assembly and an apparatus for modification.

## Background Art

Since the metal or metal alloy always has some defects or deficiencies in certain aspects of the properties, the introduction of new non-metallic elements on the surface of the metal or metal alloy is often used to improve certain aspects of the properties. For example, the introduction of nitrogen elements on the surface of the metal or non-metal may improve its hardness, abrasion resistance, corrosion resistance, etc. The hardness of the surface of the workpiece after nitriding is high, and the surface layer of the workpiece is in a compressive stress state, which significantly enhances the abrasion resistance and fatigue resistance of the workpiece and improves the scratch resistance and corrosion resistance of the surface of the workpiece. A chemical conversion film containing FeS or $FeS + FeS_2$ may be formed on a surface layer of an iron and steel workpiece after the sulfurizing treatment on the surface of the iron and steel workpiece, which may effectively reduce the friction coefficient of the surface of the iron and steel workpiece during working and improve the scratch resistance and seizure resistance.

Oxygen permeating on the surface of the titanium and titanium alloy may improve the abrasion resistance of the workpiece.

However, some currently disclosed methods for introducing non-metallic elements into the workpiece have a relatively long preparation time and are only suitable for the processing of certain workpieces with smooth surfaces and simple structures. However, for some workpieces with complicated structures and deep holes, it is difficult to ensure that the introduced elements in various parts of the workpieces have relatively high uniformity. For example, the vascular stent is in the shape of a long tube, with a relatively complicated structure. The vascular stent needs to be delivered to the lesion site through the vessel and expanded to a size corresponding to the diameter of the vessel at the lesion site so as to resist the inward contraction force of the inner wall of the vessel. Therefore, the vascular stent itself needs to have good plasticity and post-expansion capacity so that in the process of expansion, abnormal situations such as fractures cannot occur. In addition, the vascular stent needs to have a strong radial support capacity. Otherwise, it is insufficient to expand the vessel, thus failing to achieve the corresponding therapeutic effect. This leads to very strict requirements for the processing parameters of the prefabricated members or semi-finished products in various production processes of the vascular stent, especially for the processing parameters in the process of matrix modification. It is necessary to ensure the processing efficiency as well as the properties of the processed products to meet the requirements, thereby reducing the subsequent medical risks to zero as far as possible.

Commonly used metal workpiece modification may be performed by introducing non-metallic elements such as N, S, or C into the metal workpiece to improve the overall property of the metal workpiece. However, some existing conventional or traditional methods tend to result in a great difference in the contents of non-metallic elements of the inner wall and the outer wall as well as the two ends and the middle portion of the metal workpiece. For example, in the conventional non-metal introduction method, the temperature of the gas stream is uneven. In the nitriding process of the metal workpiece, there are a warm gas stream which has been heated and a cold gas stream with a relatively low temperature in the generation device. When the two gas streams are simultaneously present in the generation device, the cold gas stream will sink below due to the higher density of the cold gas stream than that of the hot gas stream. The hot gas stream contains more decomposed atomic elements than the cold gas stream. Thus, the atomic element content in the gas stream of the upper half section of the metal workpiece is relatively high, and the atomic element content in the gas stream of the lower half section is relatively low so that the content of the non-metallic element permeated in the upper half section of the metal workpiece is relatively high, while the content of the non-metallic element permeated in the lower half section is relatively low, and finally the contents of the non-metallic element permeated in the upper and lower parts of the metal workpiece is obviously uneven. In addition, due to the precise and complicated structure of some metal workpieces, especially for some metal workpieces having a tubular structure or a complicated inner structure, when using the conventional nitriding method, the amount of gas entering the inner wall of the metal workpiece is relatively small and cannot be supplemented by flow so that the content of non-metallic elements of the inner wall of the metal workpiece is relatively small, while the content of non-metallic elements of the outer wall is relatively high. The uneven content of the non-metallic elements of the inner wall and the outer wall as well as the upper part and the lower part of the metal workpiece may easily lead to inconsistent properties of various parts of the final modified metal workpiece so that there are problems in the quality of the final finished metal workpiece during use. For example, when the finished metal workpiece is a medical instrument, it is easy to occur medical accidents after being implanted into the human body, which will cause secondary damage to the patient.

With regard to the above-mentioned metal workpiece with a complicated structure and a deep hole, the non-metal permeating uniformity of the metal workpiece in the length direction is difficult to achieve using the existing non-metal permeating apparatus, which easily results in that the content of the non-metallic element at one end of the prefabricated member is much higher than that at the other end. Moreover, since the chamber of the above-mentioned metal workpiece is relatively thin, and the inner diameter of the chamber is generally 0.5-10 mm, it is difficult for the existing non-metal permeating apparatus to have a uniform non-metal permeating effect on the inner wall and the outer wall of the metal workpiece, which easily results in that the content of the non-metallic element of the outer wall of the prefabricated pipe is much higher than that of the inner wall. In addition, the existing non-metal permeating apparatus cannot meet the large-batch non-metal permeating treatment of metal workpieces.

**Summary of the Invention**

In view of the above-mentioned technical problems, the technical solutions of the present invention provide a method for integrally modifying a metal workpiece, and a gas guide assembly and an apparatus for modification. The method for integrally modifying a metal workpiece is simple in operation, easy to be industrialized, and relatively suitable for the processing of a workpiece with a relatively high requirement for the uniformity of mechanical properties of various parts, i.e., the processing of a workpiece with a relatively high requirement for the uniformity of the content of the permeated non-metallic elements. The method provided by the present invention has the advantages of simple operation, high efficiency of element permeation, safe and environmentally friendly preparation process (no toxic by-products are generated, and no toxic or highly polluting substances are used), and low cost. The modified products have the advantages of uniform distribution of newly introduced elements in any part of the workpiece, small grain size, and good mechanical properties. The gas guide assembly of the present invention is applied to the modification of a metal workpiece, and particularly to the non-metal permeating modification of the metal workpiece, so that the non-metallic gas can be preheated to a reaction temperature and then discharged from a gas guide pipe, and the gas can maintain a stable temperature and decomposition rate and a suitable flow rate, thereby improving the uniformity of the distribution of non-metallic elements after the non-metal permeating treatment of the metal workpiece. The apparatus of the present invention may be configured for large-batch non-metal permeating treatment of the metal workpieces and has a relatively good non-metal permeating treatment effect.

According to a first aspect of the present invention, there is provided a method for integrally modifying a metal workpiece, including the steps of: placing the metal workpiece in a gas stream for modification, and cooling a modified metal workpiece, wherein in the step of modification, the gas stream is preheated to a near-modification temperature, and then flows onto a surface of a to-be-modified metal workpiece.

In the present invention, the temperature of the preheated gas stream is controlled, a temperature difference between the temperature of the gas stream and the metal workpiece is reduced as much as possible, and a concentration difference between atomic non-metallic elements generated after the decomposition of the preheated gas stream and the existing gas stream near the metal workpiece is further reduced so that the content or concentration of the atomic non-metallic elements in the preheated gas stream and the temperature of the gas stream are as close as possible to those in the gas stream near the metal workpiece, thereby ensuring that an introduction rate of the non-metallic elements into various parts of the metal workpiece and the finally introduced content are as uniform as possible.

According to a second aspect of the present invention, there is provided a gas guide assembly, applied to metal workpiece modification, including:

a gas guide pipe, a relational expression between a length of the gas guide pipe and an inner radius of the gas guide pipe being: $E = LR^2 + A$;

wherein L is the length of the gas guide pipe; R is the inner radius of the gas guide pipe; E and A are constants, $0 \leq A \leq 5$ cm$^3$, and $0.3$ cm$^3 \leq E \leq 400$ cm$^3$;

wherein the gas guide assembly is capable of preheating a gas stream to a near-modification temperature and then flowing onto a surface of the metal workpiece.

In the present invention, the gas guide assembly includes a gas guide pipe having a relational expression between a length L and an inner radius R being $E=LR^2+A$, wherein $0 \leq A \leq 5$ cm$^3$ and $0.3$ cm$^3 \leq E \leq 400$ cm$^3$. The values of the length and the inner radius of the gas guide pipe may be set through the above-mentioned formula to control the preheating time of the gas in the gas guide pipe, and the gas may be sufficiently preheated by the gas guide pipe to the near-modification temperature and then discharged from the gas guide pipe so that the gas has a stable temperature and a stable decomposition rate, and the non-metal permeating of the metal workpiece is relatively uniform in the length direction. In addition, the flow rate of the gas after flowing out of the gas guide pipe may also be controlled through the above-mentioned relational expression. The flow rate of the gas is controlled within a reasonable range so that the gas can sufficiently contact with the metal workpiece, thereby avoiding the inefficiency and material waste caused by insufficient contact with the metal workpiece due to a too fast flow rate and avoiding uneven non-metal permeating of the metal workpiece in the length direction caused by premature and complete decomposition due to a too slow flow rate.

According to a third aspect of the present invention, there is provided an apparatus, including:

a reaction tool, including a heating portion and a reaction portion, where the heating portion is provided outside the reaction portion, and the reaction portion has a reaction cavity, and the reaction portion is arranged with a sample inlet communicated with the reaction cavity;

a cover body, configured to block the sample inlet, where the cover body is arranged with a gas inlet hole in a penetrating manner; and

the foregoing gas guide assembly, where an end of a gas guide pipe of the gas guide assembly close to the cover body is communicated with the gas inlet hole of the cover body; or the foregoing non-metal permeating gas guide assembly further including a gas shunting member, where an end of the gas guide pipe away from the gas shunting member of the gas guide assembly is communicated with the gas inlet hole of the cover body.

The apparatus proposed in the present invention includes a reaction tool, a cover body, and a gas guide pipe. The reaction tool includes a heating portion and a reaction portion, where the heating portion is provided outside the reaction portion, and the reaction portion has a reaction cavity, and the reaction portion is arranged with a sample inlet communicated with the reaction cavity. The cover body is configured to block the sample inlet and arranged with a gas inlet hole in a penetrating manner. An end of the gas guide pipe close to the cover body is connected to a hole wall of the gas inlet hole. Thus, the heating portion may heat the reaction portion and maintain the temperature to provide conditions under which a non-metal permeating reaction occurs. The metal workpiece enters the reaction cavity through the sample inlet of the reaction portion, and the reaction cavity provides a place for the non-metal permeating reaction. The cover body blocks the sample inlet, and the non-metallic gas may enter the reaction cavity through the gas inlet hole which is arranged by the cover body in a penetrating manner. The gas guide pipe is connected to the hole wall of the gas inlet hole, and the non-metallic gas can be preheated to the reaction temperature by the gas guide pipe and then discharged from the gas guide pipe to contact the metal workpiece to be permeated with non-metals. In this case, the non-metallic gas preheated and discharged by the gas guide pipe has a relatively stable temperature and decomposition rate and a suitable flow rate, which can improve the uniformity of a surface compound layer of the metal workpiece after the non-metal permeating treatment in the length direction. If a non-metal permeating apparatus includes a gas shunting member, the non-metallic gas flowing out of the gas guide pipe may flow into a main portion of the gas shunting member and further flow out from branch portions communicated with the main portion. The branch portions may shunt the non-metallic gas flowing out of the gas guide pipe, thereby improving the effect of the diffusion of the non-metallic gas in different directions. Thus, more non-metallic gases can enter the chamber of the metal workpiece (pipe), thereby improving the non-metal permeating effect of the inner wall of the prefabricated member (pipe), and improving the non-metal permeating uniformity of the inner wall and the outer wall of the metal workpiece (pipe).

**Brief Description of the Drawings**

Various other advantages and benefits will become apparent to a person skilled in the art upon reading the following detailed description of the preferred embodiments. The drawings are only for the purpose of illustrating the preferred embodiments and are not to be construed as limiting the present invention. Moreover, throughout the accompanying drawings, the same components are indicated by the same reference numerals.

FIG. 1 is a perspective view of an example of a non-metal permeating gas guide assembly of the present invention;

FIG. 2a is a cross-sectional view of the non-metal permeating gas guide assembly of FIG. 1;

FIG. 2b is a cross-sectional view of another example of a non-metal permeating gas guide assembly of the present invention;

FIGS. 3a-3c are schematic diagrams of multiple examples of a non-metal permeating apparatus of the present invention;

FIG. 4a is a cross-sectional view of a part of a structure of an example of a non-metal permeating apparatus of the present invention;

FIG. 4b is an enlarged view of A of the non-metal permeating apparatus shown in FIG. 4a;

FIG. 5a is a cross-sectional view of a part of a structure of another example of a non-metal permeating apparatus of the present invention;

FIG. 5b is an enlarged view of B of the non-metal permeating apparatus shown in FIG. 5a;

FIG. 6 is a diagram of metallographic structures of an iron pipe in example 3 after nitriding and annealing in a self-made nitriding furnace, wherein FIG. 6a) is a picture of the metallographic structure of a head portion of the iron pipe in example 3, FIG. 6b) is a picture of the metallographic structure of a middle portion of the iron pipe in example 3, and FIG. 6c) is a picture of the metallographic structure of a tail portion of the iron pipe in example 3; and

FIG. 7 is a diagram of metallographic structures of an iron pipe in comparative example 1 after nitriding in a well nitriding furnace, wherein FIG. 7a) is a picture of the metallographic structure of a head portion of the iron pipe in comparative example 1, FIG. 7b) is a picture of the metallographic structure of a middle portion of the iron pipe in comparative example 1, and FIG. 7c) is a picture of the metallographic structure of a tail portion of the iron pipe in comparative example 1.

## Detailed Description of the Invention

Hereinafter, exemplary embodiments of the present invention will be described in more detail with reference to the accompanying drawings. While exemplary embodiments of the present invention have been illustrated in the accompanying drawings, it is to be understood that the present invention may be embodied in various forms and should not be construed as limited to the embodiments set forth herein. On the contrary, these embodiments are provided to enable a thorough understanding of the present invention and to convey the scope of the present invention completely to a person skilled in the art. For example, the examples are illustrated using nitriding of a vascular stent as an example. However, this does not represent that the technical solutions of the present invention are only suitable for the vascular stent, nor does it represent that the technical solutions of the present invention are only suitable for nitriding, and permeating other non-metallic elements are also suitable. It should be noted that several variations and improvements made by a person skilled in the art based on the present inventive concept fall within the scope of the present invention. The reagents or instruments used are conventional products that are commercially available through regular channels without specifying the manufacturer.

The technical solution of the present invention provides a method for integrally modifying a metal workpiece, belonging to methods for permeating non-metallic elements and including the steps of: placing the metal workpiece in a gas stream for modification, and cooling a modified metal workpiece, wherein in the step of modification, the gas stream is preheated to a near-modification temperature, and then flows onto a surface of a to-be-modified metal workpiece. That is, in the technical solution provided by the present invention, the gas stream is sufficiently preheated as it flows onto the surface of the to-be-modified metal workpiece. The closer the temperature of the preheated gas stream to the temperature at which the metal workpiece is integrally modified, the more favorable is the uniform distribution of newly introduced elements in the metal.

In the present invention, the manner and position of preheating the gas stream are not limited. The gas stream may be preheated outside a modification generation device and then flow into the modification generation device, or may be preheated after flowing into the modification generation device. However, regardless of the manner, the gas stream must have been preheated and fully decomposed when reaching the surface of the to-be-modified metal workpiece.

In the above-mentioned technical solution of the present invention, when the gas stream is sufficiently decomposed, a decomposition rate of the gas reaches 15%-65%.

In the present invention, the temperature of the preheated gas stream is controlled, a temperature difference between the temperature of the gas stream and the modified metal workpiece is reduced as much as possible, and a concentration difference between atomic non-metallic elements generated after the decomposition of the preheated gas stream and the existing gas stream near the modified metal workpiece is further reduced so that the content or concentration of the atomic non-metallic elements in the preheated gas stream and the temperature of the gas stream are as close as possible to those in the gas stream near the modified metal workpiece, thereby ensuring that an introduction rate of the non-metallic elements into various parts of the modified metal workpiece and the finally introduced content are as uniform as possible.

In the above-mentioned technical solution provided by the present invention, the gas stream is preheated for 5 s-120 s before flowing onto the surface of the to-be-modified metal workpiece. Further, the gas stream is preheated for 10 s-100 s. Further, the gas stream is preheated for 12 s-90 s. If the preheating time is too short, the temperature of the gas stream is too low, and a relatively cold gas impacts the surface of the metal workpiece, affecting the temperature of the surface of the workpiece and the content of atomic non-metallic elements near the workpiece, thereby adversely affecting the modification uniformity and the final properties of the metal workpiece. The gas continues to be heated after flowing to the metal surface, and the metal workpiece itself has a certain size. Therefore, the gas stream has different temperatures when it flows to different positions of the workpiece, thereby making the contents of atomic non-metallic elements near the workpiece different. The content of atomic non-metallic elements in the part with a low temperature is low, and the content of atomic non-metallic elements in the part with a high temperature is high. Meanwhile, the higher the temperature is, the faster the workpiece is modified, and the higher the permeated atomic content is. Thus, a part of the metal workpiece that the gas stream reaches first has a lower content of non-metallic elements after modification, and a part of the metal workpiece that the gas stream reaches later has a higher content of non-metallic elements after modification, resulting in an uneven content of non-metallic elements introduced into different parts of the metal workpiece. However, if the preheating time is too long, the decomposition of the gas reaches a balance basically, no new intermediate products, i.e., atomic non-metallic elements, are continuously generated, and no or only a small amount of atomic non-metallic elements exist near a to-be-modified workpiece, which is not conducive to the modification of the metal workpiece, so that the production efficiency for integrally modifying the metal workpiece is low. Meanwhile, the consumption amount of the gas increases, and the production cost increases.

According to the above-mentioned technical solution provided by the present invention, a temperature difference between the temperature of the preheated gas stream and the temperature of any part of the workpiece is within 10°C. In the present invention, the temperature of the preheated gas stream is as close as possible to the temperature of any part of the workpiece, and the temperature difference is as small as possible. In some examples, the temperature difference between the temperature of the preheated gas stream and the temperature of any part of the workpiece is within 8°C. In other examples, the temperature difference between the temperature of the preheated gas stream and the temperature of any part of the workpiece is within 5°C.

In the present invention, the preheating time and the preheating temperature of the gas stream of a non-metallic element are controlled so that the gas stream is comprehensively controlled to reach the temperature near a to-be-modified metal workpiece and the content or concentration of the atomic non-metallic element, so as to ensure that the gas stream continuously flowing onto the surface of the to-be-modified metal workpiece has the temperature and concentration as consistent as possible with the temperature of the surface of the to-be-modified metal workpiece and the concentration of the atomic non-metallic element. Thus, the temperature of the gas stream reaching various parts of the modified workpiece and the concentration of the decomposed non-metallic atoms are as consistent as possible. That is, the uniformity or consistency of the reaction temperatures of various parts of the workpiece is ensured, and the uniformity of the concentration of the reaction raw materials in various parts of the workpiece is ensured, thereby ensuring the uniformity of the distribution of the decomposed atomic elements, and further ensuring the uniformity of the amount of the introduced non-metals in various parts of the workpiece.

According to the above-mentioned technical solution provided by the present invention, a temperature difference $\Delta T$ of the gas streams of various parts of the modified workpiece is $\leq 10°C$. Further, the temperature difference $\Delta T$ of the gas streams of various parts of the modified workpiece is $\leq 8°C$. Further, the temperature difference $\Delta T$ of the gas streams of various parts of the modified workpiece is $\leq 6°C$. The temperature difference of various parts of the modified workpiece is related to the temperature of the gas itself, the heating uniformity of various parts of the modified apparatus, and the size of the workpiece. The temperature of the surface of the workpiece can be effectively controlled only by fully controlling the influencing factors of the former two and combining the third term, i.e., the size of the modified workpiece.

The "temperature difference $\Delta T$ of the gas streams of various parts of the modified workpiece is $\leq 10°C$" described in the present invention refers to that the difference between the highest temperature and the lowest temperature of various parts of the modified workpiece is within 10°C.

According to the above-mentioned technical solution provided by the present invention, a flow rate of the gas stream is 0.5-28.0 cm/s. Further, the flow rate of the gas stream is 0.8-27.0 cm/s, 1.0-25 cm/s, 0.5-20 cm/s, 0.8-20 cm/s, 0.8-18 cm/s, 0.8-15 cm/s, 0.8-12 cm/s, 0.8-10 cm/s, 0.8-8 cm/s, 0.5-18 cm/s, 1.0-6.5 cm/s, and 1.0-7.5 cm/s. Further, the flow rate of the gas stream is 1.2-6 cm/s. In the present invention, the content of atomic non-metallic elements in the modified workpiece is controlled by controlling the flow rate of the gas stream. When the flow rate is too fast, the adsorption and permeation of decomposed atoms on the surface of the modified metal will be affected, and the efficiency of introducing non-metallic elements into the modified workpiece is relatively low. However, when the flow rate is too slow, due to the basically fixed decomposition rate of the gas into atomic elements under certain conditions, the sufficient flow of the gas in the modification generation device cannot be ensured so that the content of the atomic non-metallic elements on the surface of the modified workpiece cannot always be in a relatively stable and balanced state. Thus, the content of the elements introduced into various parts of the final modified workpiece is uneven. In addition, since the flow rate is too slow, the decomposition of the gas stream on the surfaces of various parts of the workpiece has reached a balanced state, and no new or few atomic non-metallic elements are generated so that the rate of modification is slow, and the efficiency of introducing non-metallic elements is low. Therefore, in the present invention, the flow rate of the gas stream is controlled within a relatively optimal range. The flow rate described herein is a flow rate of a gas stream within a vent pipe or a flow rate of a gas stream inside the workpiece during the modification process.

In the present invention, the gas flows on each surface of the modified workpiece, and especially for a non-planar workpiece, a gas stream is also introduced into the interior of the workpiece. For example, when the workpiece is a prefabricated member of a vascular stent and has other tubular structures, there is always a gas stream passing through an internal pipe of the workpiece. The flow rate of the gas stream is 0.5-28.0 cm/s. Further, the flow rate of the gas stream is 0.8-27.0 cm/s, 1.0-25 cm/s, 0.5-20 cm/s, 0.8-20 cm/s, 0.8-18 cm/s, 0.8-15 cm/s, 0.8-12 cm/s, 0.8-10 cm/s, 0.8-8 cm/s, 0.5-18 cm/s, 1.0-6.5 cm/s, and 1.0-7.5 cm/s. Further, the flow rate of the gas stream is 1.2-6 cm/s. It is further ensured that the content or concentration of the atomic non-metallic elements on various surfaces of the modified workpiece is as close or equivalent as possible or only fluctuates within a relatively small range. For example, the content or concentration of the atomic non-metallic elements differs by only 20%, 15%, or 10%. That is, in the present invention, the content or concentration difference of the atomic non-metallic elements on the surfaces of the workpiece during the whole modification process is maintained within 20%, further within 15%, still further within 10%, and even within 5%.

According to the above-mentioned technical solution provided by the present invention, the ventilation capacity of the gas stream is 1.5-6 L/h.

The non-metallic element described in the present invention includes at least one of N, C, and S. In the present invention, the non-metallic element may be one of N, C, and S, and may also be two or more of N, C, and S. For example, in some examples, C and N are permeated. In other examples, N is permeated.

The non-metallic element described in the present invention is at least one of C or N. Further, the non-metallic element described in the present invention is N. In this case, the method for integrally modifying a metal workpiece refers to a method for integrally modifying a metal workpiece through gas nitriding.

According to the above-mentioned technical solution provided by the present invention, the metal workpiece is integrally modified at a temperature of 410-950°C for 10 min-5 h. In the present invention, according to different types of non-metals introduced into the modified workpiece, the temperature range for introducing the non-metallic elements may also be slightly different. For example, when the introduced non-metallic element is N, the temperature for nitriding is 410-570°C, further, the temperature for nitriding is 450-570°C, or further, the temperature for nitriding is 490-570°C. When the introduced non-metallic element is C, the temperature for carburizing is relatively higher at 800-950°C, further, the temperature for carburizing is 850-950°C, or further, the temperature for carburizing is 850-930°C. When the introduced non-metallic element is S, the temperature for sulfurizing is 450-580°C, further, the temperature for sulfurizing is 480-580°C, or further, the temperature for sulfurizing is 500-560 °C. In the present invention, the metal workpiece is integrally modified for 10 min-5 h. Further, the metal workpiece is integrally modified for 10 min-4.5 h, 10 min-4 h, 10 min-3.5 h, and 10 min-3 h. Further, the metal workpiece is integrally modified for 10 min-2.5 h or 10 min-2 h.

According to the above-mentioned technical solution provided by the present invention, after introducing the non-metallic element, the method may further include the step of annealing. In the present invention, high-temperature annealing is performed after introducing the non-metallic element into the workpiece. The non-metallic element that has been introduced into the modified workpiece may be further distributed more uniformly through the high-temperature annealing. In the present invention, the annealing temperature is 800-1,200°C. When the non-metallic element is N, the annealing temperature is 800-1,000°C, and the annealing time is 5 min-5 h. When the non-metallic element is C, the annealing temperature is 600-1,200°C, and the annealing time is 20 min-10 h. Further, when the non-metallic element is N, the annealing temperature is 850-1,000°C, and the annealing time is 5 min-4.5 h; alternatively, the annealing temperature is 870-1,000°C, and the annealing time is 5 min-4 h. Further, when the non-metallic element is N, the annealing temperature is 901-1,000°C, and the annealing time is 5 min-3 h.

In the present invention, the "cooling" in the "steps of placing the metal workpiece in a gas stream for modification, and cooling a modified metal workpiece" refers to cooling the modified workpiece at room temperature after taking out the modified workpiece. In the present invention, cooling in the air may be conducive to more fine crystallization of the crystal form inside the workpiece (the smaller the crystal grain, the larger the grain size), thereby improving the mechanical properties of the workpiece, so that the modified workpiece has higher strength and better plasticity. In the present invention, the "cooling" only requires that the cooling temperature is near room temperature, specifically within a range of 5-40°C. Meanwhile, there is no limitation on the cooling medium. Cooling may be performed in air, water, oil, or any other medium.

According to the above-mentioned technical solution provided by the present invention, the gas stream includes at least one of $N_2$, $NH_3$, $CO_2$, $CH_4$, and $H_2S$. In some examples of the present invention, the gas stream includes one of the above-mentioned gases. For example, when the introduced element is N, the gas stream is $NH_3$. In other examples of the present invention, the gas stream includes at least two of the above-mentioned gases. For example, when C and N are introduced simultaneously, the gas stream is $NH_3$ and $CH_4$.

According to the above-mentioned technical solution provided by the present invention, the method further includes the step of performing high-temperature pre-oxidation in an air medium before introducing the non-metallic element. In the present invention, the modified workpiece is pre-oxidized in the air so that the efficiency of subsequent introduction of the non-metallic element may be sufficiently increased, and the time for subsequent modification of the workpiece may be greatly shortened.

According to the above-mentioned technical solution provided by the present invention, the pre-oxidation refers to oxidation at 410-950°C for 2-60 min. In the technical solution of the present invention, the specific temperature of the pre-oxidation may be appropriately adjusted within the above-mentioned interval according to the specific non-metallic element introduced. For example, in some examples of the present invention, when the introduced non-metallic element is N, the pre-oxidation time is 410-570°C. In other examples of the present invention, when the introduced non-metallic element is C, the pre-oxidation time is 450-950°C.

According to the above-mentioned technical solution provided by the present invention, the metal workpiece includes any one of prefabricated members or semi-finished products of a vascular stent, a non-vascular endoluminal stent, an occluder, an orthopedic implant, a spacer, an artificial vessel, a dental implant instrument, a vascular clamp, a dental implant, a respiratory implant, a gynecological implant, an andrological implant, a suture, a valve, and a metal wire; the orthopedic implant includes at least one of a bone nail, a bone plate, a joint, an intramedullary needle, an anchor, a bolt, and an interbody fusion cage. For example, in some examples of the present invention, the modified workpiece is one of a vascular stent semi-finished product, a valve semi-finished product, a bone nail semi-finished product, a bone plate semi-finished product, a joint semi-finished product, an intramedullary needle semi-finished product, an anchor semi-finished product, an interbody fusion cage semi-finished product, or a metal wire semi-finished product. In other examples of the present invention, the modified workpiece is one of a vascular stent prefabricated member, a valve prefabricated member, a bone nail prefabricated member, a bone plate prefabricated member, a joint prefabricated member, an intramedullary needle prefabricated member, an anchor prefabricated member, an interbody fusion cage prefabricated member, or a metal wire prefabricated member.

In the present invention, the prefabricated member refers to the upstream raw material of the workpiece. The prefabricated member may be an elongated prefabricated strip or rod with a length of 10-1,000 cm. Further, the prefabricated member may also have an elongated inner cavity. That is, the prefabricated member may be a prefabricated pipe with an inner diameter of 0.5-10 mm. Of course, the prefabricated member may also be a workpiece with a simple structure. For example, it may be a prefabricated block or a prefabricated sheet. For example, the prefabricated member of the vascular stent refers to the upstream raw material of the vascular stent. When the vascular stent is an iron-based vascular stent, the prefabricated member of the vascular stent is the upstream raw material iron pipe thereof. The semi-finished product refers to a semi-finished product in each processing step. Taking the vascular stent as an example, the semi-finished product of the vascular stent includes the products in all processing steps, such as polished member after polishing and galvanized member after galvanizing.

The metal workpiece of the present invention is also not limited to metal workpieces of medical instrument stents. Workpieces of other types of medical instruments, and even metal workpieces of other technical fields (such as electronic information technology, computers and automation, instruments, materials, or mechanical devices) are also suitable for non-metal permeating gas guide assembly of the present invention for non-metal permeating treatment.

The method provided by the present invention is suitable for the modification of all workpieces that have a high requirement for the introduction of elements, further suitable for the modification of iron-containing workpieces, further suitable for the modification of iron-based medical instruments, and further suitable for the modification of iron-based vascular stents.

It should be noted that the metal of the present invention may be a pure metal or an alloy. For example, in some examples of the present invention, the metal is pure iron, while in other examples, the metal is an iron alloy.

It should be specifically noted that "an alloy" in the present application refers to that the alloy contains the element. For example, the iron alloy refers to that the alloy contains an iron element. Further, "an alloy" may refer to that the mass or volume content of "an element" in the alloy is greater than or equal to 0.25%, and the remaining 99.75% of the components may be composed of any other metallic and/or non-metallic elements. For example, the iron alloy refers to an alloy in which the mass/volume content of iron in the alloy is greater than or equal to 0.25%, and so on. Further, the iron alloys in the present application include, but are not limited to, iron-manganese alloys, iron-zinc alloys, iron-magnesium alloys, iron-calcium alloys, iron-zirconium alloys, iron-manganese-carbon alloys, iron-molybdenum alloys, iron-manganese-copper alloys, iron-manganese-silicon-carbon alloys, iron-silicon-manganese alloys, iron-copper alloys, iron-copper-manganese-carbon alloys, iron-gold alloys, iron-silver alloys, iron-manganese-silver alloys, iron-magnesium alloys, iron-hydrogen alloys, iron-phosphorus alloys, iron-sulfur alloys, iron-manganese-carbon alloys, iron-boron alloys, iron-titanium alloys, and iron-titanium-carbon alloys.

Pure metal in the present application refers to that the total content of other impurities in the metal is less than or equal to 0.5 wt.%. For example, pure iron refers to that the total amount of metals and/or non-metals other than iron contained therein is less than or equal to 0.5 wt.%.

According to the above-mentioned technical solution provided by the present invention, the metal workpiece is made of pure iron or an iron alloy. Further, the modified workpiece is made of pure iron or an iron-based alloy with a carbon content of not more than 2.11 wt%.

According to the method for integrally modifying the workpiece provided by the above-mentioned technical solution of the present invention, the workpiece may be an iron-containing pipe having an inner diameter of 0.3 mm-12.0 mm. Further, the workpiece is an iron-containing pipe having an inner diameter of 0.5 mm-12.0mm. Further, the workpiece is an iron-containing pipe having an inner diameter of 0.5 mm-10.0 mm. In some examples of the present invention, the workpiece has a tubular structure with a very small inner diameter, and it is difficult for the gas stream to flow into the pipe so that uneven modification of an inner wall of the pipe tends to occur, resulting in uneven modification of the workpiece, thereby causing uneven mechanical properties of the workpiece easily. In some examples of the present invention, the inner diameter of the workpiece is 0.5 mm, 1 mm, 1.5 mm, 2 mm, 2.5 mm, 3 mm, 3.5 mm, 4 mm, 4.5 mm, 5 mm, 5.5 mm, 6 mm, 6.5 mm, 7 mm, 7.5 mm, 8 mm, 8.5 mm, 9 mm, 9.5 mm, or 10 mm. In other examples of the present invention, the inner diameter of the workpiece is 0.8 mm, 1.3 mm, 1.8 mm, 2.3 mm, 2.8 mm, 3.3 mm, 3.8 mm. 4.3 mm, 4.8 mm, 5.3 mm, 5.8 mm, 6.3 mm, 6.8 mm, 7.3 mm, 7.8 mm, 9.3 mm, or 9.8 mm.

According to the method for integrally modifying the workpiece provided by the above-mentioned technical solution of the present invention, a wall thickness of the workpiece is 0.05-8.0 mm. Further, the wall thickness of the workpiece is 0.1-7 mm. Further, the wall thickness of the workpiece is 0.1-6.5 mm.

According to the above-mentioned technical solution provided by the present invention, not the higher the content of the introduced non-metallic element, the better. When the content of the introduced non-metallic element is low, the modification to the workpiece is insufficient, and various mechanical properties of the workpiece are not up to the standard. When the content of the introduced non-metallic element is relatively high, the hardness and brittleness of the workpiece are increased, and the workpiece is easily fractured, thereby not meeting the requirements. Therefore, in the present invention, the content of the finally introduced non-metallic element is controlled within a relatively reasonable range through the comprehensive combination of various parameters.

According to the method for modifying the workpiece provided by the above-mentioned technical solution of the present invention, the content of the introduced non-metallic element after integrally modifying the workpiece is 300-4,000 ppm. Further, in the present invention, the content of the introduced non-metallic element after integrally modifying the workpiece is 300-3,500 ppm. Further, in the present invention, the content of the introduced non-metallic element after integrally modifying the workpiece is 300-1,500 ppm. For example, in the present invention, when the introduced non-metallic element is N, the content of the nitrogen element of the workpiece after nitriding is 300-2,500 ppm. Further, the content of the nitrogen element of the workpiece after nitriding is 300-2,000 ppm. Further, the content of the nitrogen element of the workpiece after nitriding is 300-1,500 ppm.

In the present invention, there is provided a method for modifying a workpiece, and particularly, a method for integrally modifying an iron-containing workpiece. **In** this method, other non-metallic elements are introduced into the workpiece mainly by gas nitriding or permeating other non-metallic elements, so as to sufficiently improve various mechanical properties of the workpiece.

In the present invention, the process is improved to sufficiently improve the uniformity of the temperature of the gas near the modified workpiece and to ensure the consistency of the concentration of the atomic elements decomposed near various parts of the workpiece and the reaction temperature as much as possible, thereby finally improving the stability of the elements introduced at various parts of the workpiece, ensuring the uniformity of the mechanical properties, and reducing medical accidents. In the present invention, various parts of the workpiece include a head portion of the workpiece, a tail portion corresponding to the head portion, a middle portion located in the middle of the head portion and the tail portion, and an inner wall and an outer wall of the workpiece. That is, it is necessary to ensure the uniformity of the concentration of the atomic elements in a length direction and a width direction corresponding to the length direction of the workpiece as well as the uniformity of the concentration of the atomic elements inside and outside the workpiece, thereby sufficiently ensuring the uniformity of the final mechanical properties of various parts of the workpiece.

In the present invention, a high-temperature annealing process is introduced after the modification of the workpiece so that the non-metallic elements introduced inside the workpiece are rearranged at a high temperature to further reduce the content difference of newly introduced elements at various parts so that the introduced elements at various parts are more uniform. Meanwhile, the size of the crystal form inside the metal workpiece is adjusted so that the crystal grains are smaller, and the mechanical properties of the workpiece are improved.

In the present invention, the step of pre-oxidation is introduced before the modification of the workpiece, so as to sufficiently improve the effect of subsequent modification, sufficiently reduce the modification time and the production cost, and improve the uniformity of product modification.

In the present invention, the content of non-metallic elements introduced into the workpiece is controlled within a reasonable range by comprehensively controlling the pre-oxidation temperature and time as well as the nitriding temperature and time so that the workpiece has a relatively excellent comprehensive property within this range.

In the present invention, the cooling conditions after the steps of modification and annealing are improved so that the crystal form inside the workpiece is finer, and the mechanical properties of the workpiece are better. For example, when the workpiece is a vascular stent prefabricated member, the plasticity of the subsequent finished vascular stent is better, and the mechanical properties are stronger. That is, the post-expansion capacity of the vascular stent is stronger so that the possibility of fractures during the post-expansion of the vascular stent is almost impossible. Meanwhile, the radial support force for the vessel is stronger, and the vessel can be effectively supported.

In the present invention, the indicator of the uniformity of the nitrogen content is obtained through a Vickers hardness measurement indicator. The higher the nitrogen content, the greater the hardness, whereas the lower the nitrogen content, the smaller the hardness. The uniformity of nitriding of a cross section of the iron pipe may be characterized by the nitrogen content.

In the present invention, the "head portion" of the modified workpiece refers to a part of the modified workpiece close to a vent, the "tail portion" of the modified workpiece refers to a part of the modified workpiece away from the vent, and the "middle portion" of the modified workpiece is located in the middle of the head portion and the tail portion of the modified workpiece.

The present invention also provides a gas guide assembly, which may be applied to modification of a metal workpiece, and particularly to non-metal permeating modification by placing the metal workpiece in a gas stream.

The gas guide assembly of the present invention is illustrated with the example of performing non-metal permeating modification on the metal workpiece.

The non-metal permeating gas guide assembly may guide a non-metallic gas. The non-metallic gas may be a gas nitriding medium, such as ammonia, ammonia/nitrogen, or a mixture of hydrogen/nitrogen, or may be a gas carburizing medium, a mixture of gas carburizing medium/nitriding medium, or a gas sulfurizing medium. There are a variety of metal workpieces applicable to the non-metal permeating gas guide assembly of the present invention for non-metal permeating treatment, such as those described above, which will not be repeated here.

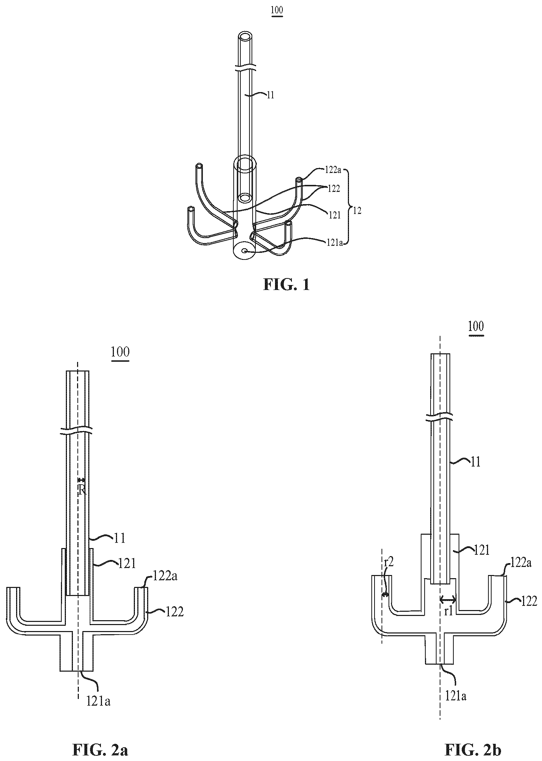

Referring to FIGS. 1-4a and 5a, a non-metal permeating gas guide assembly 100 of the present invention may include a gas guide pipe 11. The gas guide pipe 11 may be a circular pipe, a triangular pipe, a square pipe, or a polygonal pipe. The gas guide pipe 11 may be made of high temperature resistant alloy or quartz materials with good thermal conductivity.

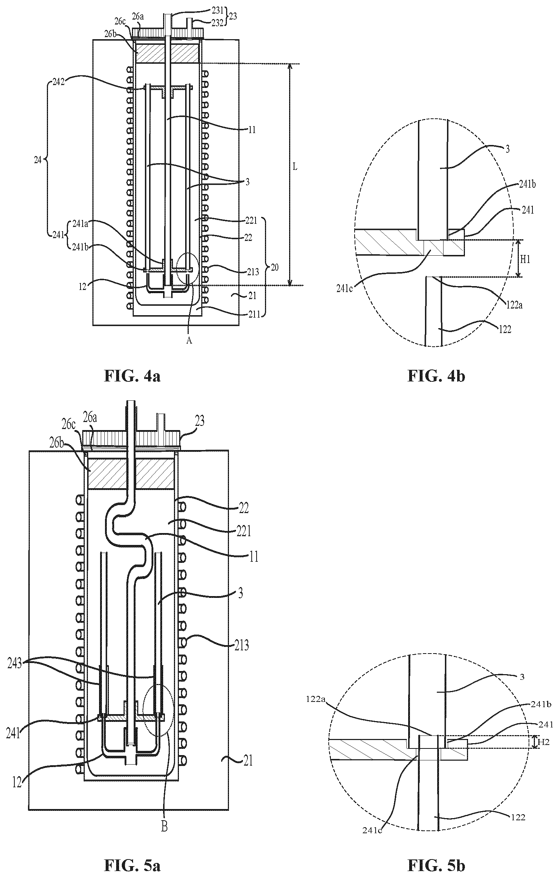

The gas guide pipe 11 may have an inner radius. The inner radius of the gas guide pipe 11 may be defined as follows. When the gas guide pipe 11 is a circular pipe, the inner radius of the gas guide pipe 11 is a radius of an inner pipe wall of the gas guide pipe 11. When the gas guide pipe 11 is not a circular pipe, such as being a square pipe, the inner radius of the gas guide pipe 11 may be a radius of a circumscribed circle of the inner pipe wall of the gas guide pipe 11. Referring to FIGS. 4a and 5a, when the gas guide assembly 100 is installed in a non-metal permeating apparatus 200, at least a part of the gas guide pipe 11 is located within a reaction cavity 221 of the non-metal permeating apparatus 200. Referring to FIG. 4a, a length L of the gas guide pipe 11 may be defined as a length of the gas guide pipe 11 below a second sealing member 26b of the non-metal permeating apparatus 200.

A relational expression between the length and the inner radius of the gas guide pipe 11 may be: $E=LR^2+A$, wherein L may be the length of the gas guide pipe 11, and R may be the inner radius of the gas guide pipe 11. A may be a constant, $0 \leq A \leq 5\,cm^3$. E may also be a constant, $0.3\,cm^3 \leq E \leq 400\,cm^3$. Further, $0.5\,cm^3 \leq A \leq 4.5\,cm^3$. Further, $1\,cm^3 \leq A \leq 4\,cm^3$. A may be 0, $0.3\,cm^3$, $0.5\,cm^3$, $0.7\,cm^3$, $1\,cm^3$, $1.5\,cm^3$, $2\,cm^3$, $3\,cm^3$, $3.5\,cm^3$, $4\,cm^3$, $4.5\,cm^3$, or $5\,cm^3$. Further, $0.5\,cm^3 \leq E \leq 350\,cm^3$. Further, $1\,cm^3 \leq E \leq 300\,cm^3$. E may include, but is not limited to, $0.3\,cm^3$, $0.5\,cm^3$, $1\,cm^3$, $5\,cm^3$, $10\,cm^3$, $50\,cm^3$, $60\,cm^3$, $70\,cm^3$, $80\,cm^3$, $90\,cm^3$, $100\,cm^3$, $120\,cm^3$, $150\,cm^3$, $180\,cm^3$, $200\,cm^3$, $220\,cm^3$, $250\,cm^3$, $280\,cm^3$, $300\,cm^3$, $320\,cm^3$, $350\,cm^3$, $370\,cm^3$, $390\,cm^3$, or $400\,cm^3$.

The values of the length and the inner radius of the gas guide pipe 11 may be set through the above-mentioned formula to control the preheating time of the gas in the gas guide pipe 11, and the gas may be sufficiently preheated by the gas guide pipe 11 to the reaction temperature and then discharged from the gas guide pipe 11 so that the temperature of the gas contacting the metal workpiece 3 is stable with a stable decomposition rate, and the non-metal permeating (modification) of the metal workpiece 3 is relatively uniform in the length direction. In addition, the flow rate of the gas after flowing out of the gas guide pipe 11 may also be controlled through the above-mentioned relational expression. The flow rate of the gas is controlled within a reasonable range so that the gas can sufficiently contact with the metal workpiece 3, thereby avoiding the inefficiency and material waste caused by insufficient contact with the metal workpiece 3 due to a too fast flow rate and avoiding uneven non-metal permeating of the metal workpiece 3 in the length direction caused by premature and complete decomposition due to a too slow flow rate.

The length of the gas guide pipe 11 may be greater than or equal to 10 cm and less than or equal to 1,000 cm. Thus, the gas guide pipe 11 has a suitable length to adapt to the size of the metal workpiece 3 to be permeated with non-metals. Further, the length L of the gas guide pipe 11 meets: $15\,cm \leq L \leq 700\,cm$. Further, $18\,cm \leq L \leq 500\,cm$. Specifically,

the length of the gas guide pipe 11 may be 10 cm, 15 cm, 18 cm, 60 cm, 100 cm, 300 cm, 500 cm, 700 cm, 900 cm, or 1,000 cm.

The value of R in the above-mentioned formula may be an average value of the inner radius of the gas guide pipe 11 or a value of an inner radius of the gas guide pipe 11 in any radial section. The inner radius of the gas guide pipe 11 may gradually increase along a flow direction of the non-metallic gas. Thus, the gas guide pipe 11 can adapt relatively to the non-metallic gas that gradually increases in volume due to heating, so as to extend the service life of the gas guide pipe 11. The inner radius of the gas guide pipe 11 may also remain unchanged, gradually decrease, or alternately increase/decrease.

The wall thickness of the gas guide pipe 11 may be greater than or equal to 0.25 mm and less than or equal to 25 mm. Thus, the gas guide pipe 11 has relatively good pressure bearing capacity and heat transfer capacity for the non-metallic gas. Similarly, the above-mentioned value may be an average value of the wall thickness of the gas guide pipe 11 or a value of the wall thickness of the gas guide pipe 11 in any radial section. The wall thickness of the gas guide pipe 11 may gradually increase along the flow direction of the non-metallic gas. Thus, the pressure bearing capacity of the gas guide pipe 11 to the non-metallic gas whose volume gradually increases due to heating is increased, and the service life of the gas guide pipe 11 is extended. Further, the wall thickness of the gas guide pipe 11 may be greater than or equal to 0.5 mm and less than or equal to 22 mm. Further, the wall thickness of the gas guide pipe 11 may be greater than or equal to 1 mm and less than or equal to 18 mm. The wall thickness of the gas guide pipe 11 may also remain unchanged, gradually decrease, or alternately increase/decrease. Specifically, the wall thickness of the gas guide pipe 11 may be 0.25 mm, 0.5 mm, 1 mm, 1.5 mm, 2 mm, 3 mm, 5 mm, 8 mm, 10 mm, 15 mm, 18 mm, 20 mm, 22 mm, 24 mm, or 25 mm.

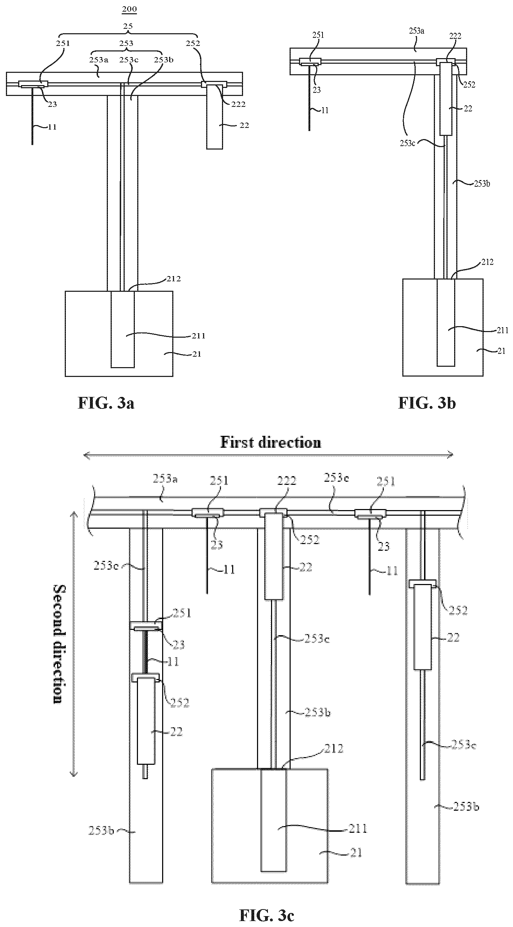

Referring to FIGS. 3a-4a, the gas guide pipe 11 may be straight, i.e., the gas guide pipe 11 may be a straight pipe. Referring to FIG. 5a, the gas guide pipe 11 may also be a bent pipe. The gas guide pipe 11 may be wavy, serrated, or spiral, etc. Thus, the shape of the gas guide pipe 11 is adapted to the length of the metal workpiece 3 and the shape of the reaction cavity 221.

Referring to FIGS. 1-2c and 4a-5b, in some examples, the non-metal permeating gas guide assembly 100 of the present invention may further include a gas shunting member 12. The gas shunting member 12 may include a main portion 121 and branch portions 122 communicated with each other. The branch portion 122 may extend radially outward along the main portion 121, and one end of the main portion 121 may be communicated with one end of the gas guide pipe 11. Thus, the non-metallic gas flowing through the gas guide pipe 11 may flow into the main portion 121 and further flow out from the branch portions 122 communicated with the main portion 121. The branch portions 122 can shunt the non-metallic gas flowing out of the gas guide pipe 11 so that the effect of the diffusion of the non-metallic gas in different directions can be improved. When the metal workpiece 3 has an elongated inner cavity, the gas shunting member 12 can increase the amount of the non-metallic gas entering the inner cavity of the metal workpiece (pipe) 3, thereby increasing the non-metal permeating uniformity of the inner wall and the outer wall of the metal workpiece (pipe) 3.

In some examples, the gas shunting member 12 and the gas guide pipe 11 may be integrally formed. That is, the main portion 121 may be an end of the gas guide pipe 11 which may be circumferentially provided with the above-mentioned branch portions 122 at intervals. The gas shunting member 12 and the gas guide pipe 11 may also be provided separately. The main portion 121 and the gas guide pipe 11 may be sleeved, welded, bonded, snapped, or screwed.

The main portion 121 and the branch portion 122 may be integrally formed. The main portion 121 and the branch portion 122 may also be detachably connected (such as welded, bonded, snapped, or screwed). The branch portion 122 may be arcuate in shape, for example, may be generally quarter circular, quarter elliptical, or quarter egg-shaped. Thus, the smooth curvature may reduce the resistance of an inner wall of the branch portion 122 to the flow of the non-metallic gas, and the non-metallic gas can have a better flow effect. The shape of the branch portion 122 may be a straight line or a fold line (such as an L shape).

The definitions of the inner radii of the main portion 121 and the branch portion 122 may refer to the above-mentioned gas guide pipe 11 and will not be repeated. Referring to FIG. 3, a relational expression between the inner radius of the main portion 121 and the inner radius of the branch portion 122 may be: $r_1 = \sqrt{nB}r_2 \pm C$, wherein $r_1$ may be the inner radius of the main portion 121, and $r_2$ may be the inner radius of the branch portion 122. n may be the number of branch portions 122, $1 \leq n \leq 10$. B may be a constant, $0.3 \leq B \leq 3$. C may be a constant, $0 \leq C \leq 5$ mm.

Further, $0.5 \leq B \leq 2.5$. Further, $0.7 \leq B \leq 2$. B may be 0.3, 0.35, 0.4, 0.5, 0.6, 0.7, 0.8, 0.9, 1.0, 1.1, 1.2, 1.3, 1.5, 1.8, 2.0, 2.2, 2.5, 2.8, or 3. Further, $0.5$ mm $\leq C \leq 4.5$ mm. Further, $1$ mm $\leq C \leq 4$ mm. C may be 0, 0.5 mm, 1 mm, 1.5 mm, 2 mm, 2.5 mm, 3 mm, 3.5 mm, 4 mm, 4.5 mm, or 5 mm.

The flow rate of the gas after flowing out of the branch portion 122 may be controlled within a reasonable range through the above-mentioned formula so that the gas can sufficiently contact with the metal workpiece 3, thereby improve the non-metal permeating uniformity of the inner wall and the outer wall of the metal workpiece 3. Specifically, when a cavity radial cross-sectional area (a single area when there is one branch portion 122, and a total area when there are multiple branch portions 122) of a gas outlet end 122a is much smaller than a cavity radial cross-sectional area of the main portion 121, the flow rate of the gas flowing out of the gas outlet end 122a is significantly larger than the flow rate of the gas in the main portion 121. When the flow rate of the gas is too fast, the gas fails to sufficiently contact with the metal workpiece 3, i.e., flowing away from the metal workpiece 3, resulting in low reaction efficiency and waste of materials. In addition, a too fast flow rate of the gas may increase the impact on the inner wall of the branch portion 122, thereby shortening the service life of the branch portion 122. When the cavity radial cross-sectional area (the same as defined above) of the gas outlet end 122a is much larger than the cavity radial cross-sectional area of the main portion 121, the flow rate of the gas flowing out of the gas outlet end 122a is relatively small, and the gas has already been decomposed completely without flowing to the other end of the metal workpiece 3, resulting in uneven non-metal permeating of the metal workpiece 3 in the length direction.

The inner radius of the branch portion 122 may be greater than or equal to 0.5 mm and less than or equal to 20 mm. Further, the inner radius of the branch portion 122 may be greater than or equal to 1 mm and less than or equal to 18 mm. Further, the inner radius of the branch portion 122 may be greater than or equal to 2 mm and less than or equal to 15 mm. Specifically, the inner radius of the branch portion 122 may be 0.5 mm, 0.8 mm, 1 mm, 1.5 mm, 2 mm, 4 mm, 5 mm, 8 mm, 10 mm, 12 mm, 14 mm, 15 mm, 18 mm, 19 mm, or 20 mm.

The inner radius of the branch portion 122 may remain unchanged, gradually increase, gradually decrease, or alternately increase/decrease. The value of the inner radius of the branch portion 122 may be the value of the inner radius of the gas outlet end of the branch portion 122 or an average value of the inner radius of the entire part of the branch portion 122. There are multiple branch portions 122. Therefore, the inner radius of the branch portion 122 may be the inner radius of a single branch portion 122 or an average value of the inner radii of multiple branch portions 122. Similarly, in the above-mentioned formula, the inner radius of the main portion 121 may be an inner radius of an end of the main portion 121 connected to the gas guide pipe 11 or an average value of the inner radius of the entire part of the main portion 121.

The gas outlet end 122a of the branch portion 122 may be provided near one end of the metal workpiece 3. The gas outlet end 122a may be directed towards one end of the prefabricated member 3. That is, an end surface of the gas outlet end 122a may be directed towards an end surface of one end of the metal workpiece 3. Thus, the amount of the non-metallic gas flowing into the inner cavity of the metal workpiece (pipe) 3 can be increased, thereby further improving the non-metal permeating treatment effect of the inner wall of the metal workpiece (pipe) 3. The end surface of the gas outlet end 122a may face the end surface of the prefabricated member 3. The end surface of the gas outlet end 122a may be parallel to the end surface of the prefabricated member 3, and the area of overlap of the two end surfaces is equal to the area of the end surface having a smaller area. The end surface of the gas outlet end 122a may also not face the end surface of the metal workpiece 3. The end surface of the gas outlet end 122a may be parallel to the end surface of the metal workpiece 3, and the two end surfaces may be at least partially staggered. Alternatively, the end surface of the gas outlet end 122a may be provided at an included angle to the end surface of the metal workpiece 3. A range of the included angle may be greater than 0 and less than 90°. Further, the range of the included angle may be greater than 0 and less than or equal to 60°. Further, the range of the included angle may be greater than 0 and less than or equal to 45°. Specifically, the range of the included angle may be 10°, 15°, 20°, 30°, 45°, 50°, 60°, 65°, 70°, 80°, or 89°.

It should be noted that when the area of the end surface of the gas outlet end 122a is greater than (e.g., greater than 30%) the area of the end surface of the metal workpiece 3, even if the included angle between the two end surfaces is 90° or even 100° (i.e., the gas outlet end 122a is not directed towards one end of the prefabricated member 3), the non-metallic gas discharged from the gas outlet end 122a can still enter the inner cavity of the metal workpiece (pipe) 3 in a sufficient amount to achieve a uniform non-metal permeating effect.

Referring to FIGS. 4a and 4b, in some examples, the gas outlet end 122a of the branch portion 122 may not extend into the end of the metal workpiece 3 close to the branch portion 122, and a distance (indicated by H1 in FIG. 4b) between the gas outlet end 122a of the branch portion 122 and the end of the metal workpiece 3 close to the branch portion 122 may be greater than or equal to 0 and less than or equal to 20 mm. Thus, the gas flowing out from the gas outlet end 122a can sufficiently enter the inner cavity of the metal workpiece (pipe) 3 so that both the inner wall and the outer wall of the metal workpiece (pipe) 3 have a relatively good non-metal permeating treatment effect. When the distance between the gas outlet end 122a of the branch portion 122 and the end of the metal workpiece 3 close to the branch portion 122 is more than 20 mm, the non-metal permeating treatment effect of the inner wall of the metal workpiece (pipe) 3 is poor. The distance between the gas outlet end 122a of the branch portion 122 and the end of the metal workpiece 3 away from the cover body 23 may be 0, 2 mm, 5 mm, 10 mm, 15 mm, 18 mm, or 20 mm.

For another example, referring to FIGS. 5a and 5b, in other examples, the gas outlet end 122a of the branch portion 122 may extend into the end of the metal workpiece (pipe) 3 close to the branch portion 122, and the distance (indicated by H2 in FIG. 5b) between the gas outlet end 122a of the branch portion 122 and the end of the metal workpiece (pipe) 3 close to the branch portion 122 may be greater than or equal to 0 and less than or equal to 10 mm. Thus, a relatively good non-metal permeating treatment effect is provided. When the extending distance is more than 10 mm, the non-metal permeating treatment effect at the overlapping part of the metal workpiece (pipe) 3 and the branch portion 122 is poor. The distance between the gas outlet end 122a of the branch portion 122 and the end of the metal workpiece (pipe) 3 away from the cover body 23 may be 0.1 mm, 0.3 mm, 0.5 mm, 0.7 mm, 1 mm, 3 mm, 5 mm, 8 mm, 9 mm. or 10 mm.

Referring to FIGS. 1-2b, an end of the main portion 121 away from the gas guide pipe 11 may be an exposed opening 121a. Thus, the non-metallic gas in the gas guide pipe 11 may flow out through the exposed opening 121a, thereby increasing the amount of the non-metallic gas contacting the outer wall of the metal workpiece (pipe) 3. Thus, the inner wall and the outer wall of the metal workpiece (pipe) 3 have a relatively uniform non-metal permeating effect. The definition of a radius of the exposed opening 121a may also refer to the above-mentioned gas guide pipe 11. The radius of the exposed opening 121a may coincide with the inner radius of the branch portion 122. At this time, the end of the main portion 121 away from the gas guide pipe 11 may be equivalent to a branch portion 122, and the gas flowing out of the exposed opening 121a has a relatively suitable flow rate, which can further improve the non-metal permeating uniformity of the inner wall and the outer wall of the metal workpiece (pipe) 3. The radius of the exposed opening 121a may coincide with the inner radius of any one of the branch portions 122, or may coincide with the average value of the inner radii of the branch portions 122. The radius of the exposed opening 121a may also be larger or smaller than the inner radius of the branch portion 122. Of course, the end of the main portion 121 away from the gas guide pipe 11 may also be sealed, and a part of the gas flowing out from the gas outlet end 122a may overflow to the outer wall of the metal workpiece (pipe) 3.

Referring to FIGS. 3a-4a and 5a, the present invention provides an apparatus 200 for modifying a metal workpiece, which is configured for modification of the metal workpiece, and particularly applicable to non-metal permeating modification of the metal workpiece. In the present invention, taking the non-metal permeating modification as an example, the non-metal permeating apparatus 200 may include a reaction tool 20, a cover body 23, and a non-metal permeating gas guide assembly 100. The reaction tool 20 may include a heating portion 21 and a reaction portion 22, and the heating portion 21 may be provided outside the reaction portion 22. The reaction portion 22 may have a reaction cavity 221 and may be arranged with a sample inlet 222 communicated with the reaction cavity 221. The cover body 23 may be configured to block the sample inlet 222 and may be arranged with a gas inlet hole 231 in a penetrating manner. The non-metal permeating apparatus 200 may include a gas guide pipe 11 of the non-metal permeating gas guide assembly 100, and an end of the gas guide pipe 11 close to the cover body 23 may be connected to a hole wall of the gas inlet hole 231. In the non-metal permeating apparatus 200, the heating portion 21 may heat the reaction portion 22 and maintain the temperature to provide conditions under which a non-metal permeating reaction occurs. The metal workpiece 3 enters the reaction cavity 221 through the sample inlet 222 of the reaction portion 22, and the reaction cavity 221 provides a place for the non-metal permeating reaction. The cover body 23 blocks the sample inlet 222, and the non-metallic gas may enter the reaction cavity 221 through the gas inlet hole 231 which is arranged by the cover body 23 in a penetrating manner. The end of the gas guide pipe 11 close to the cover body 23 is connected to the hole wall of the gas inlet hole 231. As described above, the non-metallic gas is preheated by the gas guide pipe 11 to the reaction temperature and then discharged from the gas guide pipe 11 to contact the metal workpiece 3 to be permeated with non-metals. In this case, the non-metallic gas preheated and discharged by the gas guide pipe 11 has a relatively stable temperature and decomposition rate and a suitable flow rate, thereby improving the uniformity of a surface compound layer of the metal workpiece 3 after the non-metal permeating treatment in the length direction. Therefore, the non-metal permeating apparatus 200 may have a relatively good non-metal permeating effect on the elongated metal workpiece 3. In addition, the gas guide pipe 11 is connected to the cover body 23 so that the simultaneous movement of the gas guide pipe 11 and the cover body 23 can be realized, thereby improving the efficiency and convenience of the operation.

The non-metal permeating apparatus 200 may also include a gas shunting member 12 of the non-metal permeating gas guide assembly 100, and an end of the gas guide pipe 11 away from the cover body 23 may be communicated with the main portion 121 of the gas shunting member 12. The branch portions 122 of the gas shunting member 12 can shunt the non-metallic gas flowing out of the gas guide pipe 11 so that the effect of the diffusion of the non-metallic gas in different directions may be improved, and more gas may enter the inner cavity of the metal workpiece (pipe) 3, thereby improving the uniformity of the surface compound layer on the inner wall and the outer wall of the metal workpiece (pipe) 3. Therefore, the non-metal permeating apparatus 200 may have a relatively good non-metal permeating effect on the metal workpiece (pipe) 3 having an elongated chamber.

In the above-mentioned non-metal permeating apparatus 200, the heating portion 21 and the reaction portion 22 may be integrally formed or may be detachably connected. The heating portion 21 may be provided at the periphery of the reaction portion 22. That is, the reaction portion 22 may be at least partially located in the heating portion 21. Thus, the heating cavity 211 may uniformly heat the reaction portion 22 with a relatively good heating effect. The heating portion 21 may also be provided on one side of the reaction portion 22, such as a top surface, a bottom surface, or a peripheral side.

Referring to FIGS. 3a-4a and 5a, the heating portion 21 may be a heating furnace. The heating portion 21 may have a heating cavity 211, and the reaction portion 22 may be located in the heating cavity 211 for heating. Thus, the heating portion 21 can have a relatively good heating effect on the reaction portion 22. The shape of the heating cavity 211 may be adapted to the shape of the reaction portion 22. The shape of the heating cavity 211 may be a long cylinder, and a cross section of the heating cavity 211 may be in the shape of a triangle, a circle, a square, or a polygon. The heating portion 21 may include heating members 213. The heating member 213 may be provided between a furnace wall of the heating portion 21 and the heating cavity 211. The heating member 213 may be spirally provided around the heating cavity 211, thereby improving the heating effect. The heating member 213 may also be provided on one side of the heating cavity 211, such as a top surface, a bottom surface, or a peripheral side. The heating member 213 may be a heating coil or a flame gun.

Referring to FIGS. 3a-3c, the heating portion 21 may be arranged with an opening 212 communicated with the heating cavity 211, and the reaction portion 22 may enter or move out of the heating cavity 211 through the opening 212. The opening 212 may be arranged at a top surface, a side surface, or a bottom surface of the heating portion 21. The shape of the opening 212 may be circular, polygonal, square, or irregular.

Referring to FIGS. 3a-4a and 5a, the shape of the reaction portion 22 may be a long cylinder, and a cross section of the reaction portion 22 may be in the shape of a triangle, a circle, a square, or a polygon. The reaction portion 22 may be made of high temperature resistant alloy or quartz materials. The reaction portion 22 may be made of a transparent or translucent material to facilitate viewing of the components located within the reaction portion 22 to better control the process of the non-metal permeating treatment.

Referring to FIGS. 4a and 5a, the reaction portion 22 may have a reaction cavity 221. The shape of the reaction cavity 221 may be adapted to the shape of the prefabricated member 3. The shape of the reaction cavity 221 may be a long cylinder, and a cross section of the reaction cavity 221 may be in the shape of a triangle, a circle, a square, or a polygon. **In** this way, the reaction portion 22 has a relatively good non-metal permeating effect on the prefabricated member 3.

Referring to FIGS. 3a-4a and 5a, the reaction portion 22 may be arranged with a sample inlet 222 communicated with the reaction cavity 221. The sample inlet 222 may be arranged at a top surface, a side surface, or a bottom surface of the heating portion 21. The shape of the sample inlet 222 may be circular, polygonal, square, or irregular.

Referring to FIGS. 3a-4a and 5a, the cover body 23 may be configured to block the sample inlet 222. The cover body 23 may have a plate shape, and the shape of the cover body 23 may be adapted to the shape of the sample inlet 222. For example, the cover body 23 may be a circular plate, a square plate, a polygonal plate, or an irregular plate. The cover body 23 may be made of alloy or quartz materials.

Referring to FIGS. 4a and 5a, the cover body 23 may be arranged with a gas inlet hole 231 in a penetrating manner. The shape of the gas inlet hole 231 may be circular, square, polygonal, or irregular. An end of the gas guide pipe 11 close to the cover body 23 may be communicated with the gas inlet hole 231 of the cover body 23. When an end of the gas guide pipe 11 is connected to the gas shunting member 12, the end of the gas guide pipe 11 away from the gas shunting member 12 may be communicated with the gas inlet hole 231. Specifically, an end of the gas guide pipe 11 may be connected to the hole wall of the gas inlet hole 231. The gas inlet hole 231 may be a through hole. The hole wall of the gas inlet hole 231 may extend outside the cover body 23 and may extend towards a direction away from the reaction portion 22 and/or a direction close to the reaction portion 22 so that the connection effect between the hole wall of the gas inlet hole 231 and the gas guide pipe 11 can be improved. The gas inlet hole 231 may not extend outside the cover body 23. An end of the gas guide pipe 11 connected to the cover body 23 may protrude out of the gas inlet hole 231 or may be located in the gas inlet hole 231. The gas guide pipe 11 and the gas inlet hole 231 may be welded so that the gas guide pipe 11 and the gas inlet hole 231 are connected with good sealing. The gas guide pipe 11 and the gas inlet hole 231 may also be sleeved, bonded, snapped, or screwed.

Referring to FIGS. 4a and 5a, in some examples, the cover body 23 may also be arranged with an air outlet hole 232 in a penetrating manner. The shape of the air outlet hole 232 may be circular, square, polygonal, or irregular. The air outlet hole 232 may be a through hole, and a hole wall of the air outlet hole 232 may extend towards the direction away from the reaction portion 22. Thus, a delivery pipe for delivering the exhaust gas may be conveniently connected to the hole wall of the air outlet hole 232. The hole wall of the air outlet hole 232 may not extend outside the cover body 23 or may extend towards the direction close to the reaction portion 22. In other examples, the air outlet hole 232 may be arranged by the reaction portion 22 and communicated with the reaction cavity 221.

The detachable connection of the heating portion 21, the reaction portion 22, and the cover body 23 can improve the efficiency and effect of the non-metal permeating treatment. Specifically, referring to FIGS. 3a-4a and 5a, the reaction portion 22, the cover body 23, the gas guide pipe 11, and the metal workpieces 3 may be assembled outside the reaction cavity 221 and prepared before processing in the process of heating to the reaction temperature by the heating portion 21. The assembly of the cover body 23 and the reaction portion 22 may be achieved by moving the cover body 23 towards the sample inlet 222, by moving the reaction portion 22 to make the sample inlet 222 close to the cover body 23, or by moving the cover body 23 and the reaction portion 22 together towards each other. After the reaction is completed, the reaction portion 22, the cover body 23, the gas guide pipe 11, and the metal workpieces 3 may be moved out of the heating cavity 211 for cooling. Meanwhile, the heating portion 21 may perform a non-metal permeating reaction of the next batch of metal workpieces 3. Thus, multiple processes may be performed simultaneously and independently, and the non-metal permeating apparatus 200 may be configured for large-batch non-metal permeating treatment of metal workpieces 3. In addition, when the heating portion 21 reaches the reaction temperature, the assembled reaction portion 22, cover body 23, gas guide pipe 11, and metal workpieces 3 are placed into the heating cavity 211. The reaction cavity 221 is rapidly heated, the non-metallic gas discharged from the gas guide pipe 11 has been preheated to the reaction temperature by the gas guide pipe 11, and the gas has a stable temperature and decomposition rate and a suitable flow rate, thereby having a relatively good non-metal permeating treatment effect.

Referring to FIGS. 4a and 5a, as described above, the non-metal permeating apparatus 200 of the present invention may include a fixing member 24. The fixing member 24 may be connected to the gas guide pipe 11 and configured to fix the metal workpiece 3. Thus, the cover body 23, the gas guide pipe 11, the fixing member 24, and the metal workpiece 3 may be moved together so that the efficiency and convenience of the operation can be improved. The fixing member 24 may be connected to one end of the gas guide pipe 11 (or the metal workpiece 3) or connected to the middle portion of the gas guide pipe 11 (or the metal workpiece 3).

The fixing member 24 may be made of high temperature resistant alloy or quartz materials. The fixing member 24 may include fixing sub-members (described in more detail below). There may be one or at least two fixing sub-members, and at least two fixing sub-members may be provided axially along the gas guide pipe 11 at intervals. The at least two fixing sub-members may be fixing plates, fixing rods, fixing grooves, or fixing frame, etc. The structures of the at least two fixing sub-members may be the same or different.

Referring to FIGS. 4a and 5a, when the non-metal permeating apparatus 200 includes the gas shunting member 12, the fixing member 24 can direct the end of the prefabricated member (pipe) 3 away from the cover body 23 towards the gas outlet end 122a of the branch portion 122. Thus, the gas flowing out from the gas outlet end 122a can directly flow into the inner cavity of the metal workpiece (pipe) 3 so that the amount of the gas flowing into the inner cavity of the metal workpiece (pipe) 3 is increased, and the non-metal permeating effect of the inner wall of the metal workpiece (pipe) 3 can be improved, thereby improving the non-metal permeating uniformity of the inner wall and the outer wall of the metal workpiece (pipe) 3.

Referring to FIGS. 4a and 5a, in some examples, the fixing member 24 may include a first fixing sub-member 241, as described above. The first fixing sub-member 241 may be connected to the end of the gas guide pipe 11 away from the cover body 23 or connected to the middle portion of the gas guide pipe 11. The first fixing sub-member 241 may be a fixing plate arranged with a fixing hole 241a in a penetrating manner, and the outer wall of the gas guide pipe 11 may be connected to a hole wall of the fixing hole 241a. The fixing hole 241a may be a fixing through hole, thereby improving the connection strength of the gas guide pipe 11 and the first fixing sub-member 241. The hole wall of the fixing hole 241a may extend in a direction towards the cover body 23 or in a direction away from the cover body 23. One end of the gas guide pipe 11 may or may not protrude out of the fixing hole 241a. The gas guide pipe 11 and the hole wall of the fixing hole 241a may be welded, sleeved, snapped, bonded, or screwed.

Referring to FIGS. 4a-5b, the first fixing sub-member 241 may be arranged with a fixing groove 241b. The fixing groove 241b may fix an end of the prefabricated member 3 away from the cover body 23. The fixing groove 241b may be formed by a surface of the first fixing sub-member 241 close to the cover body 23 being recessed towards a direction away from the cover body 23. That is, a notch of the fixing groove 241b may be directed towards the cover body 23. Thus, the above arrangement can prevent the fixing groove 241b from taking up additional space to obstruct the flow of the non-metallic gas so that the non-metal permeating treatment effect can be improved. The shape of the fixing groove 241b may be adapted to the prefabricated member 3. The fixing groove 241b may abut or snap with the prefabricated member 3. The fixing groove 241b may be arranged with a gas passing hole 241c in a penetrating manner at the groove bottom, and the gas may enter the prefabricated member 3 through the gas passing hole 241c. A radius of the gas passing hole 241c may be larger than, equal to, or smaller than the inner radius of the gas outlet end 122a of the branch portion 122. The shape of the gas passing hole 241c may be circular, square, triangular, waist-shaped, polygonal, or irregular. There may be one or more gas passing holes 241c. The fixing groove 241b may also be formed by extending the surface of the first fixing sub-member 241 close to the cover body 23 towards the direction of the cover body 23. That is, the fixing groove 241b is protruded from the surface of the first fixing sub-member 241 close to the cover body 23. The fixing groove 241b and the first fixing sub-member 241 may also be detachably connected, such as bonded, welded, or snapped. The first fixing sub-member 241 may also fix the middle portion of the prefabricated member 3, the fixing groove 241b may be an installation through groove, and the prefabricated member 3 may pass through the fixing groove 241b.

Referring to FIG. 4a, in some examples, the fixing member 24 may also include a second fixing sub-member 242. The second fixing sub-member 242 may be connected to the gas guide pipe 11 in the manner with reference to above-mentioned the first fixing sub-member 241. The second fixing sub-member 242 may also be a fixing plate. When the first fixing sub-member 241 fixes the end of the metal workpiece 3 away from the cover body 23, the second fixing sub-member 242 may fix the end of the metal workpiece 3 close to the cover body 23. Thus, only two ends of the metal workpiece 3 need to be cut for subsequent processing, thereby reducing the complexity of subsequent processing of the metal workpiece 3. The second fixing sub-member 242 may also be connected to the middle portion of the metal workpiece 3. The second fixing sub-piece 242 may abut against the metal workpiece 3. The second fixing sub-member 242 may be arranged with a through hole in a penetrating manner, and the metal workpiece 3 may pass through the through hole and partially abut against a hole wall of the through hole. A diameter of the through hole may be significantly larger than an outer diameter of the metal workpiece 3, and the non-metallic gas may flow into a gap between the through hole and the metal workpiece 3, thereby achieving a relatively good non-metal permeating effect. The diameter of the through hole may also be slightly larger than the outer diameter of the metal workpiece 3. The second fixing sub-member 242 is provided to improve the stability of the elongated metal workpiece 3 placed in the reaction cavity 221.

Referring to FIG. 5a, in other examples, the fixing member 24 may include a first fixing sub-member 241 and a third fixing sub-member 243. The third fixing sub-member 243 may be a fixing frame with two open ends or a fixing cylinder with multiple holes arranged on a peripheral wall in a penetrating manner. One end of the third fixing sub-member 243 close to the first fixing sub-member 241 may be connected to the first fixing sub-member 241, and an end surface of the third fixing sub-member 243 may be opposite to the notch of the fixing groove 241b. The third fixing sub-member 243 is similarly provided to improve the stability of the metal workpiece 3 placed in the reaction cavity 221.

As described above, the cover body 23 may move towards the reaction portion 22, and the reaction portion 22 may move towards the cover body 23 and the heating portion 21. There are various ways to drive the movement of the cover body 23 and the reaction portion 22. For example, the movement may be driven manually by hands. For another example, the movement may be driven by cylinders. Specifically, there may be two cylinders, and driving shafts of the two cylinders may be connected to the cover body 23 and the reaction portion 22, respectively. For another example, the movement may be driven by a motor. In some examples, the motor may be connected to mechanical arms. There may be two mechanical arms, and the two mechanical arms are connected to the cover body 23 and the reaction portion 22, respectively. The mechanical arm may include multiple sub-arms connected together through mechanical joints, and at least one sub-arm may swing around the mechanical joint on different planes (such as a horizontal plane and a vertical plane) and may include a gripping portion configured to grip/release the cover body 23 and the reaction portion 22. In other examples, the motor may be connected to sliding blocks. There may be two sliding blocks, and the two sliding blocks are connected to the cover body 23 and the reaction portion 22, respectively. The sliding blocks may drive the cover body 23 and the reaction portion 22 to move by sliding along guide rails.

Referring to FIGS. 3a-3c, the non-metal permeating apparatus 200 of the present invention may further include a driving device 25. The driving device 25 may include a first driving portion 251. A side (end) of the first driving portion 251 close to the cover body 23 may be connected to the cover body 23. The first driving portion 251 and the cover body 23 may be welded, screwed, snapped, or bonded. The driving device 25 may further include a second driving portion 252 connected to the reaction portion 22. The second driving portion 252 and the reaction portion 22 may be welded, screwed, snapped, or bonded. As described above, the driving device 25 may include a cylinder, and the first driving portion 251 and the second driving portion 252 may be driving shafts connected to the cylinder. The driving device 25 may include a motor, and the first driving portion 251 and the second driving portion 252 may be mechanical arms or sliding blocks connected to the motor. The first driving portion 251 can drive the cover body 23 to enter or move out of the reaction cavity 221 through the sample inlet 222. The second driving portion 252 can drive the reaction portion 22 to move so that the sample inlet 222 can approach or move away from the cover body 23, and the reaction portion 22 can enter or move out of the heating cavity 211 through the opening 212.

Referring to FIGS. 3a-3c, in some examples, the driving device 25 may also include a guide rail 253. The guide rail 253 may include a first sub-portion 253a extending along a first direction and a second sub-portion 253b extending along a second direction. An included angle between the first direction and the second direction may be greater than 0 and less than or equal to 90°. Referring to FIG. 3c, in some examples, the first direction may be parallel to a horizontal direction, and the second direction may be perpendicular to the horizontal direction. There may be multiple second sub-portions 253b, and multiple second sub-portions 253b may be provided along the first direction at intervals. The guide rail 253 may include sliding grooves 253c, which may extend along preset directions. The preset directions may be extending directions of the guide rail 253. That is, the preset directions may include the first direction and the second direction. The first driving portion 251 and the second driving portion 252 may be sliding blocks and are slidably connected to the sliding grooves 253c, respectively.

The non-metal permeating apparatus 200 of the present invention may also include a sealing structure. Referring to FIGS. 4a and 5a, the non-metal permeating apparatus 200 of the present invention may include a first sealing member 26a. When the cover body 23 blocks the sample inlet 222, the first sealing member 26a may seal a gap between the cover body 23 and the sample inlet 222 to prevent external air from entering the reaction cavity 211 of the reaction portion 22, thereby achieving a relatively good non-metal permeating effect. The first sealing member 26a may be provided on the cover body 23, for example, on a side of the cover body 23 close to the sample inlet 222 or on a peripheral side of the cover body 23. The first sealing member 26a may be provided at the sample inlet 222 of the reaction portion 22. There may be two first sealing members 26a. The two first sealing members 26a may be provided on the cover body 23 and the reaction portion 22, respectively, or the two first sealing members 26a may be provided on the same component. There may be two or more first sealing members 26a. The first sealing member 26a may be made of at least one of high temperature resistant rubber, refractory cotton, asbestos, quartz fiber, or aerogel.

Referring to FIGS. 4a and 5a, the non-metal permeating apparatus 200 of the present invention may include a second sealing member 26b. When the cover body 23 blocks the sample inlet 222, the second sealing member 26b may be located in the reaction cavity 221 to seal the reaction cavity 221. Thus, the reaction cavity 221 can have a relatively good heat preservation effect so that a relatively good non-metal permeating effect can be achieved. The second sealing member 26b may be connected to the gas guide pipe 11 and may be provided close to the cover body 23. When the cover body 23 drives the gas guide pipe 11 into the reaction cavity 221, the gas guide pipe 11 may drive the second sealing member 26b, the fixing member 24, and the metal workpiece 3 fixed on the fixing member 24 together into the reaction cavity 221. Thus, the second sealing member 26b does not obstruct the metal workpiece 3 from entering the reaction cavity 221. The shape of the second sealing member 26b may be adapted to the shape of the reaction cavity 221. For example, the second sealing member 26b may be a circular block, a square block, or a polygonal block.

The second sealing member 26b may be a refractory brick. The thickness of the second sealing member 26b may be greater than or equal to 20 mm and less than or equal to 150 mm. Further, the thickness of the second sealing member 26b may be greater than or equal to 30 mm and less than or equal to 140 mm. Further, the thickness of the second sealing member 26b may be greater than or equal to 40 mm and less than or equal to 130 mm. The thickness of the second sealing member 26b may be 20 mm, 30 mm, 32 mm, 40 mm, 45 mm, 55 mm, 65 mm, 75 mm, 90 mm, 110 mm, 120 mm, 130 mm, 135 mm, 140 mm, or 150 mm. The second sealing member 26b may be prepared from at least one of silicon, carbon, zirconium, aluminum, aluminum silicate, magnesium, magnesium-calcium, magnesium-chromium, magnesium-aluminum, or magnesium-silicon refractory materials.

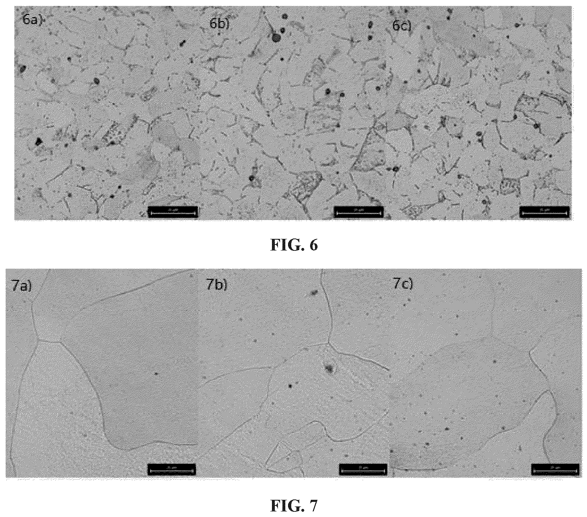

Referring to FIGS. 4a and 5a, the non-metal permeating apparatus 200 of the present invention may include a third sealing member 26c. When the reaction portion 22 is located in the heating cavity 211, the third sealing member 26c may be located at the opening 222 to seal the heating cavity 211. Referring to FIGS. 5 and 7, in some examples, the third sealing member 26c may be provided on a part of the heating cavity 211 close to the opening 222. The third sealing member 26c may fill a gap between the heating cavity 211 and the reaction portion 22 to seal the heating cavity 211. In other examples, the third sealing member 26c may be provided outside the heating cavity 211. An outer wall of the reaction portion 22 may be provided with a boss (not shown) close to the sample inlet 222 and extending circumferentially along the reaction portion 22, and the third sealing member 26c may be located between the heating portion 21 and the boss to seal the heating cavity 211. There may be one third sealing member 26c provided on the reaction portion 22 or the heating portion 21. There may also be two third sealing members 26c provided on the reaction portion 22 and the heating portion 21, respectively, or on the same component. There may be two or more third sealing members 26c. The second sealing member 26b may also be made of at least one of high temperature resistant rubber, refractory cotton, asbestos, quartz fiber, or aerogel.

In the technical solution provided by the present invention, a non-metal permeating method implemented by the above-mentioned non-metal permeating apparatus 200 may include the steps of:

S0, heating the heating portion 21 to a non-metal permeating reaction temperature; S1, placing the gas guide pipe 11, the fixing member 24 (if any), and the metal workpiece 3 into the reaction cavity 221 of the reaction portion 22, blocking the reaction cavity 221 by the cover body 23, and moving the assembled cover body 23, reaction portion 22, gas guide pipe 11, fixing member 24 (if any), and metal workpiece 3 into the heating cavity to perform a non-metal permeating reaction; S2, after the non-metal permeating reaction is finished, evacuating the reaction cavity 221 or filling the reaction cavity 221 with an inert gas (such as argon, helium, or nitrogen), and setting the temperature of the heating cavity 211 to an annealing temperature to perform an annealing reaction; S3, after the annealing reaction is completed, moving the assembled reaction portion 22, cover body 23, gas guide pipe 11, fixing member 24 (if any), and metal workpiece 3 out of the heating cavity for cooling, and after the cooling is completed, taking the non-metal permeated metal workpiece 3 out from the reaction cavity 221.

Unless otherwise specified, the terms "within", "above", "below", and "beyond" in the present invention include the numerical values themselves. For example, "the decomposition rate of the preheated gas stream is within 10% of the decomposition rate of the existing gas stream on the surface of the to-be-modified workpiece" refers to that the decomposition rate of the preheated gas stream differs by 10% or less from the decomposition rate of the existing gas stream on the surface of the to-be-modified workpiece. That is, 10% is included.

It should be understood that the terms used herein are for the purpose of describing particular exemplary embodiments only and are not intended to form a limitation. As used herein, the singular forms "a", "an", and "the" are intended to include the plural forms as well, unless the context clearly indicates otherwise. The terms "comprising", "including", "containing", and "having" are inclusive, and therefore specify the presence of stated features, steps, operations, elements, and/or components, but do not preclude the presence or addition of one or more other features, steps, operations, elements, components, and/or combinations thereof. The method steps, processes, and operations described herein are not to be construed as necessarily requiring that they be performed in the particular order described or illustrated, unless the order of performance is explicitly stated. It should also be understood that additional or alternative steps may be used.

**Test methods**

The test methods for various parameters in the present invention are as follows.

1. Testing of Vickers hardness:

The samples are polished after being embedded in resin, and then tested according to the standard "GB/T 4340.1-2012 Metallic materials-Vickers hardness test".

2. Testing of nitrogen content:

The samples are tested according to the standard "GB/T 20124-2006 Steel and iron-Determination of nitrogen content-Thermal conductimetric method after fusion in a current of inert gas".

3. Testing of grain size:

The samples are polished after being embedded in resin, etched with a 4% nitric acid alcohol solution for 10 s, and then photographed and observed under a metallographic microscope. The pictures are tested according to the standard "GB/T 6394-2017 Determination of estimating the average grain size".

4. Testing of various dimensions of iron pipe:

The diameter of the iron pipe is measured using a laser diameter measuring instrument, the wall thickness is measured using a three-dimensional microscope, and the length is measured using a meter ruler or a vernier caliper.

**Example 1**

Referring to FIGS. 3a, 3b, 4a, and 5b, when a single batch of prefabricated members 3 were nitrided, the non-metal permeating apparatus 200 could be used as follows:

S0, heating the heating cavity 211 to a non-metal permeating reaction temperature and maintaining the temperature;

S1, penetrating the prefabricated members 3 into the second fixing sub-member 242 and the first fixing sub-member 241;

S2, driving the cover body 23 by the first driving portion 251 to move from a first position to a second position;

S3, introducing ammonia into the reaction cavity 221 through the gas guide pipe 11, and discharging the air in the reaction cavity 221 through the air outlet hole 232;

S4, driving the cover body 23 by the first driving portion 251, and driving the reaction portion 22 by the second driving portion 252 to move from the second position to a third position simultaneously;

S5, preheating ammonia through the gas guide pipe 11, then discharging into the reaction cavity 221, and decomposing at a high temperature to obtain active nitrogen ions; performing nitriding on an outer pipe wall and an inner pipe wall of the prefabricated member 3;

S6, after the nitriding reaction is finished, evacuating the reaction portion 22;

S7, changing the temperature of the heating cavity 211 to an annealing temperature and maintaining the temperature to perform annealing of the nitriding reaction;

S8, after the annealing is completed, driving the cover body 23 by the first driving portion 251, and driving the reaction portion 22 by the second driving portion 252 to move from the third position to the second position simultaneously for cooling;

S9, after the cooling is completed, stopping evacuating, and venting;

S10, driving the cover body 23 by the first driving portion 251 to move from the second position to the first position; and

S11, taking out the nitrided prefabricated member 3.

**Example 2**

Referring to FIGS. 3c, 4a, and 5a, when multiple batches of prefabricated members 3 were nitrided, the non-metal permeating apparatus 200 could be specifically used as follows:

S1, performing nitriding on the first batch of prefabricated members 3, the process being the same as S0-S5 in example 1;

S2, preparing a second batch of prefabricated members 3 during the nitriding of the first batch of prefabricated members 3, the preparation process being the same as S0-S3 in example 1;

S3, after the nitriding reaction is finished, annealing and cooling the first batch of prefabricated members 3, the process being the same as S6-S8 in example 1;

S4, performing nitriding on the second batch of prefabricated members 3, the process being the same as S4-S5 in example 1;

S5, preparing a third batch of prefabricated members 3 during the nitriding of the second batch of prefabricated members 3, and paying attention to whether the cooling of the first batch of prefabricated members 3 is completed;

S6, after the cooling of the first batch of prefabricated members 3 is completed, stopping evacuating, venting, and then taking out the nitrided prefabricated member 3, the process being the same as S9-S11 in example 1; and

S7, the processes of the second and subsequent batches of prefabricated members 3 repeating the process of the first batch of prefabricated members 3.

**Example 3**

In this example, the method for integrally modifying the metal workpiece was implemented using the non-metal permeating apparatus 200. Taking a single batch as an example, the selected metal workpiece was an iron pipe. The iron pipe was drawn from an original pipe blank and was in a cold-drawn state. The iron pipe had an outer diameter of $\varphi$6 mm, a wall thickness of 0.5 mm, and an inner diameter of 5 mm. A longitudinal metallographic structure of the iron pipe was deformed, and a nitrogen flow was provided using ammonia for nitriding.

1. The iron pipe was cleaned to remove oil, rinsed with alcohol to remove residual water on the surface, dried using an air gun, and placed vertically in a nitriding apparatus 200 for fixation.

2. The apparatus was started for pre-oxidation treatment. The pre-oxidation was performed at 570°C for 30 min, and the iron pipe was cooled in the air after oxidation was completed.

3. Nitriding parameters were set. The flow capacity of ammonia was 6 L/h, the flow rate was 8 cm/s, the preheating time was 5 s, and the decomposition rate of ammonia was 20%. The nitriding time was 2 h, and the nitriding temperature was 570°C. A head-tail temperature difference of the iron pipe was 10°C. After the nitriding was finished, the iron pipe was taken out and cooled in the air to obtain a nitrided iron pipe sample.

4. The nitrided iron pipe was annealed in a high-temperature annealing furnace at an annealing temperature of 800°C for 10 min. After the annealing was completed, the iron pipe was cooled in the air, and the nitriding and annealing of the iron pipe were completed.

A grinding wheel cutting machine was used to cut each of the head, middle, and tail of the iron pipe sample by approximately 500 g to prepare nitrogen content test samples, cut each of the head, middle, and tail of the iron pipe sample by 1 cm to prepare hardness test samples, and cut each of the head, middle, and tail of the iron pipe sample by 1 cm to prepare metallographic samples.

An ONH analyzer was used to measure nitrogen content data, and a Vickers hardness tester was used to test hardness data of the 3 hardness samples. The 3 metallographic samples were polished through an automatic polishing machine and etched using a 4% nitric acid alcohol solution for 10 s to obtain the metallographic samples. The metallographic structure was observed in a metallographic microscope, and the metallographic structure pictures and grain size data were as follows.

| Parts | | Head | Middle | Tail |
|---|---|---|---|---|
| Vickers hardness HV/0.05 | Inner wall | 330.4 | 340.1 | 337.4 |
| | Core | 334 | 341.6 | 338.5 |
| | Outer wall | 335.9 | 338.9 | 341.7 |
| Iron pipe temperature | | 565.2 | 571.5 | 575.2 |
| Nitrogen content/ppm | | 1258 | 1296 | 1305 |
| Grain size | | 7.8 | 7.6 | 8 |

**Example 4**

In this example, the selected iron pipe was drawn from an original pipe blank and was in a cold-drawn state. The iron pipe had an outer diameter of φ6 mm, a wall thickness of 0.5 mm, and an inner diameter of 5 mm. A longitudinal metallographic structure of the iron pipe was deformed, and a nitrogen flow was provided using ammonia for nitriding.

1. The iron pipe was cleaned to remove oil, rinsed with alcohol to remove residual water on the surface, dried using an air gun, and placed vertically in a nitriding apparatus 200.

2. The apparatus was started for pre-oxidation treatment. The pre-oxidation was performed at 470°C for 2 min, and the iron pipe was cooled in the air after oxidation was completed.

3. Nitriding parameters were set. The flow capacity of ammonia was 1.5 L/h, the flow rate of ammonia was 0.5 cm/s, the preheating time was 120 s, and the decomposition rate of ammonia was 65%. The nitriding time was 10 min, and the nitriding temperature was 470°C. A head-tail temperature difference of the iron pipe was 0.8°C. After the nitriding was finished, the iron pipe was taken out and cooled in the air to obtain a nitrided iron pipe sample.

4. The nitrided iron pipe was annealed in a high-temperature annealing furnace at an annealing temperature of 1,200°C for 2 h. After the annealing was completed, the iron pipe was cooled in the air, and the nitriding and annealing of the iron pipe were completed.

A grinding wheel cutting machine was used to cut each of the head, middle, and tail of the iron pipe sample by approximately 500 g to prepare nitrogen content test samples, cut each of the head, middle, and tail of the iron pipe sample by 1 cm to prepare hardness test samples, and cut each of the head, middle, and tail of the iron pipe sample by 1 cm to prepare metallographic samples.

An ONH analyzer was used to measure nitrogen content data, and a Vickers hardness tester was used to test hardness data of the 3 hardness samples. The 3 metallographic samples were polished through an automatic polishing machine and etched using a 4% nitric acid alcohol solution for 10 s to obtain the metallographic samples. The metallographic structure was observed in a metallographic microscope, and the metallographic structure pictures and grain size data were as follows.

| Parts | | Head | Middle | Tail |
|---|---|---|---|---|
| Vickers hardness HV/0.05 | Inner wall | 265.6 | 260.3 | 264.2 |
| | Core | 261.4 | 259.8 | 263.3 |
| | Outer wall | 260.7 | 264.2 | 263.7 |
| Iron pipe temperature | | 469.2 | 470.1 | 469.3 |
| Nitrogen content/ppm | | 658 | 702 | 672 |
| Grain size | | 5.1 | 5.9 | 5.4 |

**Example 5**

In this example, the selected iron pipe was drawn from an original pipe blank and was in a cold-drawn state. The iron pipe had an outer diameter of φ6 mm, a wall thickness of 0.5 mm, and an inner diameter of 5 mm. A longitudinal metallographic structure of the iron pipe was deformed, and a nitrogen flow was provided using ammonia for nitriding.

1. The iron pipe was cleaned to remove oil, rinsed with alcohol to remove residual water on the surface, dried using an air gun, and placed vertically in a nitriding apparatus 200.

2. The apparatus was started for pre-oxidation treatment. The pre-oxidation was performed at 540°C for 5 min, and the iron pipe was cooled in the air after oxidation was completed.

3. Nitriding parameters were set. The flow capacity of ammonia was 4.7 L/h, the flow rate of ammonia was 4 cm/s, the preheating time was 40 s, and the decomposition rate of ammonia was 42%. The nitriding time was 30 min, and the nitriding temperature was 540°C. A head-tail temperature difference of the iron pipe was 3.9°C. After the nitriding was finished, the iron pipe was taken out and cooled in the air to obtain a nitrided iron pipe sample.

4. The nitrided iron pipe was annealed in a high-temperature annealing furnace at an annealing temperature of 950°C for 30 min. After the annealing was completed, the iron pipe was cooled in the air, and the nitriding and annealing of the iron pipe were completed.

A grinding wheel cutting machine was used to cut each of the head, middle, and tail of the iron pipe sample by approximately 500 g to prepare nitrogen content test samples, cut each of the head, middle, and tail of the iron pipe sample by 1 cm to prepare hardness test samples, and cut each of the head, middle, and tail of the iron pipe sample by 1 cm to prepare metallographic samples.

An ONH analyzer was used to measure nitrogen content data, and a Vickers hardness tester was used to test hardness data of the 3 hardness samples. The 3 metallographic samples were polished through an automatic polishing machine and etched using a 4% nitric acid alcohol solution for 10 s to obtain the metallographic samples. The metallographic structure was observed in a metallographic microscope, and the metallographic structure pictures and grain size data were as follows.

| Parts | | Head | Middle | Tail |
|---|---|---|---|---|
| Vickers hardness HV/0.05 | Inner wall | 305.2 | 304.1 | 305.3 |
| | Core | 308.5 | 306.2 | 308.4 |
| | Outer wall | 304.6 | 307.3 | 305.5 |
| Iron pipe temperature | | 538.7 | 541.2 | 542.6 |
| Nitrogen content/ppm | | 1103 | 1002 | 1080 |
| Grain size | | 7.5 | 7.4 | 7.2 |

**Example 6**

In this example, the selected iron pipe was drawn from an original pipe blank and was in a cold-drawn state. The iron pipe had an outer diameter of φ6 mm, a wall thickness of 0.5 mm, and an inner diameter of 5 mm. A longitudinal metallographic structure of the iron pipe was deformed, and a nitrogen flow was provided using ammonia for nitriding.

1. The iron pipe was cleaned to remove oil, rinsed with alcohol to remove residual water on the surface, dried using an air gun, and placed vertically in a nitriding apparatus 200 for fixation.

2. The apparatus was started for pre-oxidation treatment. The pre-oxidation was performed at 560°C for 25 min, and the iron pipe was cooled in the air after oxidation was completed.

3. Nitriding parameters were set. The flow capacity of ammonia was 5.5 L/h, the flow rate of ammonia was 7.5 cm/s, the preheating time was 15 s, and the decomposition rate of ammonia was 26%. The nitriding time was 90 min, and the nitriding temperature was 560°C. A head-tail temperature difference of the iron pipe was 7.2°C. After the nitriding was finished, the iron pipe was taken out and cooled in the air to obtain a nitrided iron pipe sample.

4. The nitrided iron pipe was annealed in a high-temperature annealing furnace at an annealing temperature of 850°C for 20 min. After the annealing was completed, the iron pipe was cooled in the air, and the nitriding and annealing of the iron pipe were completed.

A grinding wheel cutting machine was used to cut each of the head, middle, and tail of the iron pipe sample by approximately 500 g to prepare nitrogen content test samples, cut each of the head, middle, and tail of the iron pipe sample by 1 cm to prepare hardness test samples, and cut each of the head, middle, and tail of the iron pipe sample by 1 cm to prepare metallographic samples.

An ONH analyzer was used to measure nitrogen content data, and a Vickers hardness tester was used to test hardness data of the 3 hardness samples. The 3 metallographic samples were polished through an automatic polishing machine and etched using a 4% nitric acid alcohol solution for 10 s to obtain the metallographic samples. The metallographic structure was observed in a metallographic microscope, and the metallographic structure pictures and grain size data were as follows.

| Parts | | Head | Middle | Tail |
|---|---|---|---|---|
| Vickers hardness HV/0.05 | Inner wall | 320.4 | 327.1 | 322.6 |
| | Core | 319.8 | 321.2 | 325.4 |
| | Outer wall | 323.9 | 318.7 | 324.9 |
| Iron pipe temperature | | 553.2 | 559.4 | 560.3 |
| Nitrogen content/ppm | | 1231 | 1209 | 1215 |
| Grain size | | 7.5 | 7.4 | 7.4 |

**Example 7**

In this example, the selected iron pipe was drawn from an original pipe blank and was in a cold-drawn state. The iron pipe had an outer diameter of $\varphi$6 mm, a wall thickness of 0.5 mm, and an inner diameter of 5 mm. A longitudinal metallographic structure of the iron pipe was deformed, and a nitrogen flow was provided using ammonia for nitriding.

1. The iron pipe was cleaned to remove oil, rinsed with alcohol to remove residual water on the surface, dried using an air gun, and placed vertically in a nitriding apparatus 200 for fixation.

2. The apparatus was started for pre-oxidation treatment. The pre-oxidation was performed at 550°C for 20 min, and the iron pipe was cooled in the air after oxidation was completed.

3. Nitriding parameters were set. The flow capacity of ammonia was 4.5 L/h, the flow rate of ammonia was 6 cm/s, the preheating time was 30 s, and the decomposition rate of ammonia was 39%. The nitriding time was 60 min, and the nitriding temperature was 550°C. A head-tail temperature difference of the iron pipe was 4.7°C. After the nitriding was finished, the iron pipe was taken out and cooled in the air to obtain a nitrided iron pipe sample.

4. The nitrided iron pipe was annealed in a high-temperature annealing furnace at an annealing temperature of 901°C for 30 min. After the annealing was completed, the iron pipe was cooled in the air, and the nitriding and annealing of the iron pipe were completed.

A grinding wheel cutting machine was used to cut each of the head, middle, and tail of the iron pipe sample by approximately 500 g to prepare nitrogen content test samples, cut each of the head, middle, and tail of the iron pipe sample by 1 cm to prepare hardness test samples, and cut each of the head, middle, and tail of the iron pipe sample by 1 cm to prepare metallographic samples.

An ONH analyzer was used to measure nitrogen content data, and a Vickers hardness tester was used to test hardness data of the 3 hardness samples. The 3 metallographic samples were polished through an automatic polishing machine and etched using a 4% nitric acid alcohol solution for 10 s to obtain the metallographic samples. The metallographic structure was observed in a metallographic microscope, and the metallographic structure pictures and grain size data were as follows.

| Parts | | Head | Middle | Tail |
|---|---|---|---|---|
| Vickers hardness HV/0.05 | Inner wall | 310.5 | 318.9 | 314.2 |
| | Core | 317.1 | 311.9 | 312.8 |
| | Outer wall | 316.4 | 314.2 | 313.8 |
| Iron pipe temperature | | 545.6 | 549.8 | 550.3 |
| Nitrogen content/ppm | | 1115 | 1142 | 1194 |
| Grain size | | 7.4 | 7 | 7.3 |

**Example 8**

In this example, the selected iron pipe was drawn from an original pipe blank and was in a cold-drawn state. The iron pipe had an outer diameter of φ6 mm, a wall thickness of 0.3 mm, and an inner diameter of 5.4 mm. A longitudinal metallographic structure of the iron pipe was deformed, and a nitrogen flow was provided using ammonia for nitriding.

1. The iron pipe was cleaned to remove oil, rinsed with alcohol to remove residual water on the surface, dried using an air gun, and placed vertically in a nitriding apparatus 200 for fixation.

2. The apparatus was started for pre-oxidation treatment. The pre-oxidation was performed at 530°C for 10 min, and the iron pipe was cooled in the air after oxidation was completed.

3. Nitriding parameters were set. The flow capacity of ammonia was 4 L/h, the flow rate of ammonia was 4 cm/s, the preheating time was 40 s, and the decomposition rate of ammonia was 44%. The nitriding time was 45min, and the nitriding temperature was 530°C. Ahead-tail temperature difference of the iron pipe was 2.1°C. After the nitriding was finished, the iron pipe was taken out and cooled in the air to obtain a nitrided iron pipe sample.

4. The nitrided iron pipe was annealed in a high-temperature annealing furnace at an annealing temperature of 940°C for 40 min. After the annealing was completed, the iron pipe was cooled in the air, and the nitriding and annealing of the iron pipe were completed.

A grinding wheel cutting machine was used to cut each of the head, middle, and tail of the iron pipe sample by approximately 500 g to prepare nitrogen content test samples, cut each of the head, middle, and tail of the iron pipe sample by 1 cm to prepare hardness test samples, and cut each of the head, middle, and tail of the iron pipe sample by 1 cm to prepare metallographic samples.

An ONH analyzer was used to measure nitrogen content data, and a Vickers hardness tester was used to test hardness data of the 3 hardness samples. The 3 metallographic samples were polished through an automatic polishing machine and etched using a 4% nitric acid alcohol solution for 10 s to obtain the metallographic samples. The metallographic structure was observed in a metallographic microscope, and the metallographic structure pictures and grain size data were as follows.

| Parts | | Head | Middle | Tail |
|---|---|---|---|---|
| Vickers hardness HV/0.05 | Inner wall | 302.4 | 305.1 | 306.9 |
| | Core | 301.9 | 304.2 | 300.8 |
| | Outer wall | 305.4 | 301.8 | 302.6 |
| Iron pipe temperature | | 528.4 | 530.1 | 530.5 |
| Nitrogen content/ppm | | 1023 | 1012 | 1024 |
| Grain size | | 7.1 | 6.8 | 7.4 |

**Example 9**

In this example, the selected iron pipe was drawn from an original pipe blank and was in a cold-drawn state. The iron pipe had an outer diameter of φ6 mm, a wall thickness of 0.5 mm, and an inner diameter of 5 mm. A longitudinal metallographic structure of the iron pipe was deformed, and a nitrogen flow was provided using ammonia for nitriding.

1. The iron pipe was cleaned to remove oil, rinsed with alcohol to remove residual water on the surface, dried using an air gun, and placed vertically in a nitriding apparatus 200 for fixation.

2. The apparatus was started for pre-oxidation treatment. The pre-oxidation was performed at 520°C for 4 min, and the iron pipe was cooled in the air after oxidation was completed.

3. Nitriding parameters were set. The flow capacity of ammonia was 3.5 L/h, the flow rate of ammonia was 3 cm/s, the preheating time was 50 s, and the decomposition rate of ammonia was 48%. The nitriding time was 30 min, and the nitriding temperature was 520°C. A head-tail temperature difference of the iron pipe was 1.4°C. After the nitriding was finished, the iron pipe was taken out and cooled in the air to obtain a nitrided iron pipe sample.

4. The nitrided iron pipe was annealed in a high-temperature annealing furnace at an annealing temperature of 960°C for 50 min. After the annealing was completed, the iron pipe was cooled in the air, and the nitriding and annealing of the iron pipe were completed.

A grinding wheel cutting machine was used to cut each of the head, middle, and tail of the iron pipe sample by approximately 500 g to prepare nitrogen content test samples, cut each of the head, middle, and tail of the iron pipe sample by 1 cm to prepare hardness test samples, and cut each of the head, middle, and tail of the iron pipe sample by 1 cm to prepare metallographic samples.

An ONH analyzer was used to measure nitrogen content data, and a Vickers hardness tester was used to test hardness data of the 3 hardness samples. The 3 metallographic samples were polished through an automatic polishing machine and etched using a 4% nitric acid alcohol solution for 10 s to obtain the metallographic samples. The metallographic structure was observed in a metallographic microscope, and the metallographic structure pictures and grain size data were as follows.

| Parts | | Head | Middle | Tail |
|---|---|---|---|---|
| Vickers hardness HV/0.05 | Inner wall | 294.1 | 289.4 | 293.2 |
| | Core | 289.5 | 290 | 291.4 |
| | Outer wall | 293.6 | 291.8 | 292.2 |
| Iron pipe temperature | | 519.6 | 520.1 | 521 |
| Nitrogen content/ppm | | 996 | 987 | 1001 |
| Grain size | | 6.7 | 6.6 | 6.4 |

## Example 10

In this example, the selected iron pipe was drawn from an original pipe blank and was in a cold-drawn state. The iron pipe had an outer diameter of $\varphi$6 mm, a wall thickness of 0.5 mm, and an inner diameter of 5 mm. A longitudinal metallographic structure of the iron pipe was deformed, and a nitrogen flow was provided using ammonia for nitriding.

1. The iron pipe was cleaned to remove oil, rinsed with alcohol to remove residual water on the surface, dried using an air gun, and placed vertically in a nitriding apparatus 200 for fixation.

2. The apparatus was started for pre-oxidation treatment. The pre-oxidation was performed at 500°C for 2 min, and the iron pipe was cooled in the air after oxidation was completed.

3. Nitriding parameters were set. The flow capacity of ammonia was 3 L/h, the flow rate of ammonia was 1 cm/s, the preheating time was 80 s, and the decomposition rate of ammonia was 53%. The nitriding time was 25 min, and the nitriding temperature was 500°C. A head-tail temperature difference of the iron pipe was 1.2°C. After the nitriding was finished, the iron pipe was taken out and cooled in the air to obtain a nitrided iron pipe sample.

4. The nitrided iron pipe was annealed in a high-temperature annealing furnace at an annealing temperature of 1,000°C for 60 min. After the annealing was completed, the iron pipe was cooled in the air, and the nitriding and annealing of the iron pipe were completed.

A grinding wheel cutting machine was used to cut each of the head, middle, and tail of the iron pipe sample by approximately 500 g to prepare nitrogen content test samples, cut each of the head, middle, and tail of the iron pipe sample by 1 cm to prepare hardness test samples, and cut each of the head, middle, and tail of the iron pipe sample by 1 cm to prepare metallographic samples.

An ONH analyzer was used to measure nitrogen content data, and a Vickers hardness tester was used to test hardness data of the 3 hardness samples. The 3 metallographic samples were polished through an automatic polishing machine and etched using a 4% nitric acid alcohol solution for 10 s to obtain the metallographic samples. The metallographic structure was observed in a metallographic microscope, and the metallographic structure pictures and grain size data were as follows.

| Parts | | Head | Middle | Tail |
|---|---|---|---|---|
| Vickers hardness | Inner | 286.2 | 287.3 | 275.2 |
| HV/0.05 | wall | | | |
| | Core | 290 | 293.2 | 294.1 |
| | Outer wall | 289.4 | 291.2 | 291.3 |
| Iron pipe temperature | | 499.2 | 500.2 | 500.4 |
| Nitrogen content/ppm | | 894 | 863 | 901 |
| Grain size | | 6.7 | 6.2 | 6.4 |

**Example 11**

In this example, the selected iron pipe was drawn from an original pipe blank and was in a cold-drawn state. The iron pipe had an outer diameter of φ6 mm, a wall thickness of 0.5 mm, and an inner diameter of 5 mm. A longitudinal metallographic structure of the iron pipe was deformed, and a carbon flow was provided using methane for carburizing.

1. The iron pipe was cleaned to remove oil, rinsed with alcohol to remove residual water on the surface, dried using an air gun, and placed vertically in a carburizing apparatus 200 for fixation.

2. Carburizing parameters were set. The flow capacity of methane was 3 L/h, the flow rate of methane was 3 cm/s, the preheating time was 60 s, and the decomposition rate of methane was 20%. The carburizing time was 1 h, and the carburizing temperature was 950°C. A head-tail temperature difference of the iron pipe was 1°C. After the carburizing was finished, the iron pipe was taken out and cooled in the air to obtain a carburized iron pipe sample.

3. The nitrided iron pipe was annealed in a high-temperature annealing furnace at an annealing temperature of 1,000°C for 60 min. After the annealing was completed, the iron pipe was cooled in the air, and the carburizing and annealing of the iron pipe were completed.

A grinding wheel cutting machine was used to cut each of the head, middle, and tail of the iron pipe sample by approximately 500 g to prepare nitrogen content test samples, cut each of the head, middle, and tail of the iron pipe sample by 1 cm to prepare hardness test samples, and cut each of the head, middle, and tail of the iron pipe sample by 1 cm to prepare metallographic samples.

A CS analyzer was used to measure carbon content data, and a Vickers hardness tester was used to test hardness data of the 3 hardness samples. The 3 metallographic samples were polished through an automatic polishing machine and etched using a 4% nitric acid alcohol solution for 10 s to obtain the metallographic samples. The metallographic structure was observed in a metallographic microscope, and the metallographic structure pictures and grain size data were as follows.

| Parts | | Head | Middle | Tail |
|---|---|---|---|---|
| Vickers hardness HV/0.05 | Inner wall | 265.4 | 267.1 | 259.8 |
| | Core | 260.4 | 258.3 | 264.1 |
| | Outer wall | 263.7 | 264.2 | 260.8 |
| Iron pipe temperature | | 948.2 | 950.3 | 951.2 |
| C content/ppm | | 904 | 932 | 945 |
| Grain size | | 5.1 | 5.4 | 5 |

**Example 12**

In this example, the selected iron pipe was drawn from an original pipe blank and was in a cold-drawn state. The iron pipe had an outer diameter of φ6 mm, a wall thickness of 0.5 mm, and an inner diameter of 5 mm. A longitudinal metallographic structure of the iron pipe was deformed, and a nitrogen flow was provided using ammonia for nitriding.

1. The iron pipe was cleaned to remove oil, rinsed with alcohol to remove residual water on the surface, dried using an air gun, and placed vertically in a self-made nitriding furnace for fixation.

2. The apparatus was started for pre-oxidation treatment. The pre-oxidation was performed at 570°C for 50 min, and the iron pipe was cooled in the air after oxidation was completed.

3. Nitriding parameters were set. The flow capacity of ammonia was 20 L/h, the flow rate of ammonia was 28 cm/s, the preheating time was 30 s, and the decomposition rate of ammonia was 41%. The nitriding time was 5 h, and the nitriding temperature was 570°C. Ahead-tail temperature difference of the iron pipe was 0.8°C. After the nitriding was finished, the iron pipe was taken out and cooled in the air to obtain a nitrided iron pipe sample.

4. The nitrided iron pipe was annealed in a high-temperature annealing furnace at an annealing temperature of 800°C for 10 h. After the annealing was completed, the iron pipe was cooled in the air, and the nitriding and annealing of the iron pipe were completed.

A grinding wheel cutting machine was used to cut each of the head, middle, and tail of the iron pipe sample by approximately 500 g to prepare nitrogen content test samples, cut each of the head, middle, and tail of the iron pipe sample by 1 cm to prepare hardness test samples, and cut each of the head, middle, and tail of the iron pipe sample by 1 cm to prepare metallographic samples.
An ONH analyzer was used to measure nitrogen content data, and a Vickers hardness tester was used to test hardness data of the 3 hardness samples. The 3 metallographic samples were polished through an automatic polishing machine and etched using a 4% nitric acid alcohol solution for 10 s to obtain the metallographic samples. The metallographic structure was observed in a metallographic microscope, and the metallographic structure pictures and grain size data were as follows.

| Parts | | Head | Middle | Tail |
|---|---|---|---|---|
| Vickers hardness HV/0.05 | Inner wall | 324.4 | 325.6 | 326.1 |
| | Core | 325.1 | 325.4 | 326.3 |
| | Outer wall | 326.1 | 325.2 | 325.7 |
| Iron pipe temperature | | 561.5 | 562 | 562.3 |
| Nitrogen content/ppm | | 1254 | 1257 | 1266 |
| Grain size | | 7.2 | 7.4 | 7.4 |

**Example 13**

In this example, the selected iron pipe was drawn from an original pipe blank and was in a cold-drawn state. The iron pipe had an outer diameter of φ1.4 mm, a wall thickness of 0.08 mm, and an inner diameter of 1.24 mm. A longitudinal metallographic structure of the iron pipe was deformed, and a nitrogen flow was provided using ammonia for nitriding.

1. The iron pipe was cleaned to remove oil, rinsed with alcohol to remove residual water on the surface, dried using an air gun, and placed vertically in a nitriding apparatus 200 for fixation.

2. The apparatus was started for pre-oxidation treatment. The pre-oxidation was performed at 570°C for 50 min, and the iron pipe was cooled in the air after oxidation was completed.

3. Nitriding parameters were set. The flow capacity of ammonia was 20 L/h, the flow rate of ammonia was 28 cm/s, the preheating time was 30 s, and the decomposition rate of ammonia was 41%. The nitriding time was 1 h, and the nitriding temperature was 570°C. Ahead-tail temperature difference of the iron pipe was 0.8°C. After the nitriding was finished, the iron pipe was taken out and cooled in the air to obtain a nitrided iron pipe sample.

4. The nitrided iron pipe was annealed in a high-temperature annealing furnace at an annealing temperature of 800°C for 10 min. After the annealing was completed, the iron pipe was cooled in the air, and the nitriding and annealing of the iron pipe were completed.

A grinding wheel cutting machine was used to cut each of the head, middle, and tail of the iron pipe sample by approximately 500 g to prepare nitrogen content test samples, cut each of the head, middle, and tail of the iron pipe sample by 1 cm to prepare hardness test samples, and cut each of the head, middle, and tail of the iron pipe sample by 1 cm to prepare metallographic samples.

An ONH analyzer was used to measure nitrogen content data, and a Vickers hardness tester was used to test hardness data of the 3 hardness samples. The 3 metallographic samples were polished through an automatic polishing machine and etched using a 4% nitric acid alcohol solution for 10 s to obtain the metallographic samples. The metallographic structure was observed in a metallographic microscope, and the metallographic structure pictures and grain size data were as follows.

| Parts | | Head | Middle | Tail |
|---|---|---|---|---|
| Vickers hardness HV/0.05 | Inner wall | 321.4 | 323.6 | 325.1 |
| | Core | 322.3 | 322.4 | 324.3 |
| | Outer wall | 323.1 | 323.2 | 325.2 |
| Iron pipe temperature | | 561.2 | 561 | 562.0 |
| Nitrogen content/ppm | | 1230 | 1235 | 1228 |
| Grain size | | 7.3 | 7.5 | 7.3 |

**Comparative example 1**

In this example, the selected iron pipe was drawn from an original pipe blank and was in a cold-drawn state. The iron pipe had an outer diameter of $\varphi$6 mm, a wall thickness of 0.5 mm, and an inner diameter of 5 mm. A longitudinal metallographic structure of the iron pipe was deformed, and a nitrogen flow was provided using ammonia for nitriding.

1. The iron pipe was cleaned to remove oil, rinsed with alcohol to remove residual water on the surface, dried using an air gun, and placed in an automatic nitriding furnace.

2. The nitriding temperature was set to 540°C, and the flow rate of nitrogen was 8 cm/s. The iron pipe was placed horizontally, and a head-tail temperature difference of the iron pipe was 1.1°C. The nitriding was performed for 12 h, and after the nitriding was finished, the iron pipe was cooled in the air.

A grinding wheel cutting machine was used to cut each of the head, middle, and tail of the iron pipe sample by approximately 500 g to prepare nitrogen content test samples, cut each of the head, middle, and tail of the iron pipe sample by 1 cm to prepare hardness test samples, and cut each of the head, middle, and tail of the iron pipe sample by 1 cm to prepare metallographic samples.

An ONH analyzer was used to measure nitrogen content data, and a Vickers hardness tester was used to test hardness data of the 3 hardness samples. The 3 metallographic samples were polished using an automatic polishing machine and etched using a 4% nitric acid alcohol solution for 10 s to obtain the metallographic samples. The metallographic structure was observed in a metallographic microscope, and the metallographic structure pictures and grain size data were as follows.

| Parts | | Head | Middle | Tail |
|---|---|---|---|---|
| Vickers hardness HV/0.05 | Inner wall | 268.4 | 265.7 | 251.1 |
| | Core | 305.2 | 307.4 | 304.3 |
| | Outer wall | 356.4 | 368.1 | 364.2 |
| Iron pipe temperature | | 541.2 | 542.3 | 541.8 |
| Nitrogen content/ppm | | 1325 | 1298 | 1253 |
| Grain size | | 4.2 | 4.3 | 4.2 |

**Comparative example 2**

In this example, the selected iron pipe was drawn from an original pipe blank and was in a cold-drawn state. The iron pipe had an outer diameter of φ6 mm, a wall thickness of 0.5 mm, and an inner diameter of 5 mm. A longitudinal metallographic structure of the iron pipe was deformed, and a nitrogen flow was provided using ammonia for nitriding.

1. The iron pipe was cleaned to remove oil, rinsed with alcohol to remove residual water on the surface, dried using an air gun, and placed vertically in a self-made nitriding furnace.

2. The apparatus was started for pre-oxidation treatment. The pre-oxidation was performed at 540°C for 5 min, and the iron pipe was cooled in the air after oxidation was completed.

3. Nitriding parameters were set. The flow capacity of ammonia was 4.7 L/h, the flow rate of ammonia was 4 cm/s, preheating was not performed, and the decomposition rate of ammonia was 10%. The nitriding time was 30 min, and the nitriding temperature was 540°C. A head-tail temperature difference of the iron pipe was 12.9°C. After the nitriding was finished, the iron pipe was taken out and cooled in the air to obtain a nitrided iron pipe sample.

4. The nitrided iron pipe was annealed in a high-temperature annealing furnace at an annealing temperature of 950°C for 30 min. After the annealing was completed, the iron pipe was cooled in the air, and the nitriding and annealing of the iron pipe were completed.

An ONH analyzer was used to measure nitrogen content data, and a Vickers hardness tester was used to test hardness data of the 3 hardness samples. The 3 metallographic samples were polished using an automatic polishing machine and etched using a 4% nitric acid alcohol solution for 10 s to obtain the metallographic samples. The metallographic structure was observed in a metallographic microscope, and the metallographic structure pictures and grain size data were as follows.

| Parts | | Head | Middle | Tail |
|---|---|---|---|---|
| Vickers hardness HV/0.05 | Inner wall | 235.4 | 286.6 | 301.2 |
| | Core | 248.9 | 270.3 | 300.5 |
| | Outer wall | 251.2 | 281.4 | 298.7 |
| Iron pipe temperature | | 515.1 | 536.4 | 542.8 |
| Nitrogen content/ppm | | 704 | 805 | 876 |
| Grain size | | 6.2 | 6.4 | 7.1 |

**Comparative example 3**

In this example, the selected iron pipe was drawn from an original pipe blank and was in a cold-drawn state. The iron pipe had an outer diameter of φ6 mm, a wall thickness of 0.5 mm, and an inner diameter of 5 mm. A longitudinal metallographic structure of the iron pipe was deformed, and a nitrogen flow was provided using ammonia for nitriding.

1. The iron pipe was cleaned to remove oil, rinsed with alcohol to remove residual water on the surface, dried using an air gun, and placed vertically in a self-made nitriding furnace.

2. The apparatus was started, and nitriding parameters were set. The flow capacity of ammonia was 4.5 L/h, the flow rate of ammonia was 6 cm/s, the preheating time was 30 s, and the decomposition rate of ammonia was 39%. The nitriding time was 60 min, and the nitriding temperature was 550°C. A head-tail temperature difference of the iron pipe was 2.5°C. After the nitriding was finished, the iron pipe was taken out and cooled in the air to obtain a nitrided iron pipe sample.

3. The nitrided iron pipe was annealed in a high-temperature annealing furnace at an annealing temperature of 900°C for 30 min. After the annealing was completed, the iron pipe was cooled in the air, and the nitriding and annealing of the iron pipe were completed.

An ONH analyzer was used to measure nitrogen content data, and a Vickers hardness tester was used to test hardness data of the 3 hardness samples. The 3 metallographic samples were polished using an automatic polishing machine and etched using a 4% nitric acid alcohol solution for 10 s to obtain the metallographic samples. The metallographic structure was observed in a metallographic microscope, and the metallographic structure pictures and grain size data were as follows.

| Parts | | Head | Middle | Tail |
|---|---|---|---|---|
| Vickers hardness HV/0.05 | Inner wall | 273.5 | 271.2 | 269.7 |
| | Core | 269.3 | 268.4 | 270.5 |
| | Outer wall | 272.2 | 270.8 | 269.9 |
| Iron pipe temperature | | 548.4 | 549.6 | 550.9 |
| Nitrogen content/ppm | | 854 | 882 | 831 |
| Grain size | | 5.4 | 6 | 5.7 |

It can be seen from the above that the nitrogen contents in comparative example 1 and example 3 are similar. However, in example 3, the grain size is larger, and the size of the crystal grain is smaller. The reason is that the annealing process is provided in example 3. The vascular stent iron pipe prefabricated member is annealed after nitriding so that the nitrogen atoms in the prefabricated member are diffused uniformly, and the crystal grains are refined, which is reflected in the vascular stent as having high radial strength, providing good support for the vessels.

Compared with example 4, in comparative example 1, the lack of gas stream passing through the middle portion of the vascular stent prefabricated member leads to an insufficient nitrogen content on the inner wall and an excessive nitrogen content on the outer wall of the prefabricated member, showing uneven hardness on the cross section of the prefabricated member. When processed into a vascular stent, this causes the phenomenon of low inner wall hardness and high outer wall hardness. In the expansion process, the outer wall is easily cracked due to the high hardness, which eventually leads to the fracture of the vascular stent. However, in example 4, the inner and outer walls may be nitrided simultaneously due to the internal ammonia flow, showing that the hardness of the inner and outer walls is uniform, effectively improving the over-expansion property of the vascular stent.

Compared with example 5, in comparative example 2, the hardness at the head portion is generally lower than that at the tail portion. The reason is that in comparative example 2, the gas flows directly over the inner surface of the workpiece without preheating, resulting in uneven temperatures at the head portion and the tail portion of the workpiece, with a significant head-tail temperature difference. The head portion is cooled by the low-temperature gas, resulting in a lower surface temperature and less nitriding, leading to smaller hardness. However, the tail portion experiences less cooling from the gas so that it has a higher temperature, a higher nitrogen content, and a greater hardness. After cutting into the vascular stents, the vascular stent produced in the head portion will have low strength, while the vascular stent produced in the tail portion will have high strength, which cannot ensure the stability of product properties.

Compared with example 7, in comparative example 3, under the same nitriding conditions, the nitriding efficiency is greatly reduced due to the lack of a pre-oxidation step. It can be seen that the nitrogen content in comparative example 3 is much lower than that in example 7. Due to the lack of a pre-oxidation process, in order to achieve the required nitrogen content during production, it is necessary to extend the nitriding time, which greatly reduces the production efficiency.

In examples 3-10, the nitriding temperatures, nitriding time, gas flow rates, preheating time, annealing temperatures, and annealing time are different, which is reflected in the difference in the nitrogen contents in the prefabricated member. In addition, the hardness of the prefabricated member also decreases with the decrease in the nitrogen content. In these examples, it can be seen that the cross section of the prefabricated member has good uniformity. The reason is that the inner and outer walls of the vascular stent prefabricated member have good nitriding effects.

The above are only preferred embodiments of the present invention, and the scope of thepresent invention is not limited thereto. Any changes and substitutions which can be readily thought of by a person skilled in the art within the technical scope disclosed in the present invention fall within the scope of the present invention.

**Claims**

1. A method for integrally modifying a metal workpiece, comprising the steps of: placing the metal workpiece in a gas stream for modification, and cooling a modified metal workpiece, wherein in the step of modification, the gas stream is preheated to a near-modification temperature, and then flows onto a surface of the metal workpiece.

2. The method for integrally modifying a metal workpiece according to claim 1, wherein the gas stream is preheated for 5 s-120 s as flowing onto a surface of the to-be-modified metal workpiece; a difference between a temperature of a preheated gas stream and a temperature of any part of the workpiece is within 10°C; a temperature difference $\Delta T$ of various parts of the modified workpiece is $\leq$ 10°C.

3. The method for integrally modifying a metal workpiece according to claim 1, wherein a flow rate of the gas stream is 0.5-28.0 cm/s; the metal workpiece is integrally modified at a temperature of 410-950°C for 10 min-5 h; the cooling refers to cooling the modified workpiece at room temperature after taking out the modified workpiece; non-metallic elements comprise N, C, and S.

4. The method for integrally modifying a metal workpiece according to claim 1, wherein the method for integrally modifying a metal workpiece further comprises the step of annealing, wherein the annealing is performed at a temperature of 800-1,200°C for 10 min-10 h.

5. The method for integrally modifying a metal workpiece according to claim 1, wherein before modifying the workpiece, the method further comprises the step of performing high-temperature pre-oxidation in an air medium, wherein the high-temperature pre-oxidation refers to oxidation at 410-950°C for 2-60 min.

6. The method for integrally modifying a metal workpiece according to claim 1, wherein the modified workpiece comprises any one of prefabricated members or semifinished products of a vascular stent, a non-vascular endoluminal stent, an occluder, an orthopedic implant, a spacer, an artificial vessel, a dental implant instrument, a vascular clamp, a dental implant, a respiratory implant, a gynecological implant, an andrological implant, a suture, a valve, and a metal wire; the orthopedic implant comprises at least one of a bone nail, a bone plate, a joint, an intramedullary needle, an anchor, a bolt, and an interbody fusion cage.

7. The method for integrally modifying a metal workpiece according to claim 1, wherein the modified workpiece is made of pure iron or an iron alloy; the workpiece is an iron-containing pipe having an inner diameter of 0.3 mm-12.0 mm.

8. A gas guide assembly, applied to metal workpiece modification, comprising:

   a gas guide pipe, a relational expression between a length of the gas guide pipe and an inner radius of the gas guide pipe being: $E = LR^2 + A$;
   wherein L is the length of the gas guide pipe; R is the inner radius of the gas guide pipe; E and A are constants, $0 \leq A \leq 5$ cm$^3$ and 0.3 cm$^3 \leq E \leq 400$ cm$^3$;
   wherein the gas guide assembly is capable of preheating a gas stream to a near-modification temperature and then flowing onto a surface of the metal workpiece.

9. The gas guide assembly according to claim 8, wherein the length of the gas guide pipe is greater than or equal to 10 cm and less than or equal to 1,000 cm; a wall thickness of the gas guide pipe is greater than or equal to 0.25 mm and less than or equal to 25 mm.

10. The gas guide assembly according to claim 8, further comprising:

a gas shunting member, comprising a main portion and branch portions communicated with each other, wherein one end of the main portion is communicated with one end of the gas guide pipe, and the branch portion extends radially outward along the main portion.

11. The gas guide assembly according to claim 10, wherein a relational expression between an inner radius of the main portion and an inner radius of the branch portion is: $r_1=\sqrt{nB}r_2\pm C$ , wherein $r_1$ is the inner radius of the main portion; $r_2$ is the inner radius of the branch portion; n is the number of branch portions, $1\leq n\leq 10$; B is a constant, $0.3\leq B\leq 3$; C is constant, $0\leq C\leq 5$ mm.

12. The gas guide assembly according to claim 11, wherein the inner radius of the branch portion is greater than or equal to 0.5 mm and less than or equal to 20 mm.

13. The gas guide assembly according to claim 10, wherein a gas outlet end of the branch portion is directed towards an end of a prefabricated member; a distance between the gas outlet end of the branch portion and the end of the prefabricated member close to the branch portion is greater than or equal to 0 mm and less than or equal to 20 mm; or the gas outlet end of the branch portion extends into the end of the prefabricated member close to the branch portion, and the distance between the gas outlet end of the branch portion and the end of the prefabricated member close to the branch portion is greater than 0 mm and less than or equal to 10 mm.

14. The gas guide assembly according to claim 10, wherein an end of the main portion away from the gas guide pipe is an exposed opening.

15. An apparatus, applied to metal workpiece modification, comprising:

a reaction tool, comprising a heating portion and a reaction portion, wherein the heating portion is provided outside the reaction portion, and the reaction portion has a reaction cavity, and the reaction portion is arranged with a sample inlet communicated with the reaction cavity;
a cover body, configured to block the sample inlet, wherein the cover body is arranged with a gas inlet hole in a penetrating manner; and
the gas guide assembly according to claims 8 and 9, wherein an end of a gas guide pipe of the gas guide assembly close to the cover body is communicated with the gas inlet hole of the cover body; or the gas guide assembly according to any one of claims 10-14, wherein an end of the gas guide pipe away from a gas shunting member of the gas guide assembly is communicated with the gas inlet hole of the cover body.

16. The apparatus according to claim 15, further comprising:
a fixing member, connected to the gas guide pipe and configured to fix the metal workpiece.

17. The apparatus according to claim 16, wherein the fixing member comprises a first fixing sub-member and a second fixing sub-member, and the first fixing sub-member and the second fixing sub-member are configured to fix two ends of the metal workpiece, respectively.

18. The apparatus according to claim 15, further comprising:

a first sealing member, wherein when the cover body blocks the sample inlet, the first sealing member is located between the cover body and the sample inlet to sealingly connect the cover body and the sample inlet; and
a second sealing member, wherein when the cover body blocks the sample inlet, the second sealing member is located in the reaction cavity to seal the reaction cavity.

19. The apparatus according to claim 18, wherein a thickness of the second sealing member is greater than or equal to 20 mm and less than or equal to 150 mm; the second sealing member is prepared from at least one of silicon, carbon, zirconium, aluminum, aluminum silicate, magnesium, magnesium-calcium, magnesium-chromium, magnesium-aluminum, or magnesium-silicon refractory materials.

20. The apparatus according to claim 15, wherein the heating portion has a heating cavity, and the heating portion is arranged with an opening communicated with the heating cavity, and the reaction portion is able to enter or move out of the heating cavity through the opening.

**21.** The apparatus according to claim 20, further comprising:
a driving device, comprising a first driving portion and a second driving portion, wherein the first driving portion is connected to the cover body to drive the cover body to move towards/away from the sample inlet; the second driving portion is connected to the reaction portion to drive the reaction portion to move towards/away from the cover body and/or the opening.

**22.** The apparatus according to claim 21, wherein the driving device comprises a guide rail, and the guide rail comprises sliding grooves extending along preset directions; the first driving portion and the second driving portion are slidably connected to the sliding grooves, respectively.

**23.** The apparatus according to claim 20, further comprising:
a third sealing member, wherein when the reaction portion is located in the heating cavity, the third sealing member is located at the opening to seal the heating cavity.

**FIG. 1**

**FIG. 2a**

**FIG. 2b**

FIG. 3a

FIG. 3b

FIG. 3c

FIG. 4a

FIG. 4b

FIG. 5a

FIG. 5b

**FIG. 6**

**FIG. 7**

## INTERNATIONAL SEARCH REPORT

| International application No. |
|---|
| **PCT/CN2023/110535** |

| A. | CLASSIFICATION OF SUBJECT MATTER |
|---|---|

C23C8/26(2006.01)i;  C23C8/36(2006.01)i;  C23C8/22(2006.01)i;  C23C8/06(2006.01)i

According to International Patent Classification (IPC) or to both national classification and IPC

| B. | FIELDS SEARCHED |
|---|---|

Minimum documentation searched (classification system followed by classification symbols)

   IPC: C23C

Documentation searched other than minimum documentation to the extent that such documents are included in the fields searched

Electronic data base consulted during the international search (name of data base and, where practicable, search terms used)

   CNABS; CNTXT; DWPI; WPABS; USTXT; EPTXT; WOTXT; CNKI; ISI Web of Knowledge: 元心科技, 渗碳, 渗氮, 氮化, 硫化, 预热, 气体, 导气, 进气, 管, 分支, 金属, 医用, 盖, 密封, 反应, 腔, 室, 加热, 移出, Carburizing, Nitriding, Vulcanization, Preheat, gas, inlet, pipe, branch, metal, medical, cover, lid, seal, reaction, chamber, heat, remove

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP H0790541 A (DEMUTETSUKU KK) 04 April 1995 (1995-04-04) description, paragraphs 0034-0044, and figure 14 | 1-9 |
| Y | JP H0790541 A (DEMUTETSUKU KK) 04 April 1995 (1995-04-04) description, paragraphs 0034-0044, and figure 14 | 15-20, 23 |
| Y | CN 203049017 U (GAO ZHONGBAO) 10 July 2013 (2013-07-10) description, paragraphs 0007-0016, and figure 1 | 15-20, 23 |
| Y | GB 403792 A (ADOLPH WILHELM MACHLET) 04 January 1934 (1934-01-04) description, page 2, left-hand column, line 34-page 3, right-hand column, line 77 | 20, 23 |
| X | JP 2012126962 A (KOYO THERMO SYSTEM KK) 05 July 2012 (2012-07-05) description, paragraphs 0017-0027, and figure 1 | 1-9 |
| X | JP H06248416 A (DEMU TEC KK) 06 September 1994 (1994-09-06) description, paragraphs 0006-0009, and figure 1 | 1-9 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **27 November 2023** | **05 December 2023** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2023/110535**

| Patent document cited in search report | | | Publication date (day/month/year) | Patent family member(s) | Publication date (day/month/year) |
|---|---|---|---|---|---|
| JP | H0790541 | A | 04 April 1995 | None | |
| CN | 203049017 | U | 10 July 2013 | None | |
| GB | 403792 | A | 04 January 1934 | None | |
| JP | 2012126962 | A | 05 July 2012 | None | |
| JP | H06248416 | A | 06 September 1994 | None | |

Form PCT/ISA/210 (patent family annex) (July 2022)

**REFERENCES CITED IN THE DESCRIPTION**

**Patent documents cited in the description**

- CN 202210916370
- CN 202211091239